# FASCICULE DE BREVET EUROPEEN

(11) **EP 0 770 138 B1**
(45) Date de publication et mention de la délivrance du brevet: **13.02.2008**
(21) Numéro de dépôt: 95929147.7
(22) Date de dépôt: 30.08.1995
(51) Int. Cl.: C12N 15/74, C12N 15/31, C07K 14/35, C12N 15/62, A61K 39/04, A61K 39/116, C12Q 1/68, C07K 16/12, C12N 1/21

(54) **VECTEURS DE CRIBLAGE ET/OU D'EXPRESSION FONCTIONNELS CHEZ LES MYCOBACTERIES**
FUNKTIONELLES AUFSPÜREN UND/ODER EXPRESSIONSVEKTOREN BEI MYCOBAKTERIEN
MYCOBACTERIA FUNCTIONAL SCREENING AND/OR EXPRESSION VECTORS

(30) Priorité: 02.09.1994 FR 9410585
(43) Date de publication de la demande: 02.05.1997
(60) Demande divisionnaire: 03021544.6
(73) Titulaire: INSTITUT PASTEUR, 75724 Paris Cédex 15 (FR)
(72) Inventeur: GICQUEL, Brigitte, 75014 Paris (FR); LIM, Eng Mong, 75015 Paris (FR); PORTNOI, Denis, 75004 Paris (FR); BERTHET, François-Xavier, 75015 Paris (FR); TIMM, Juliano, 75011 Paris (FR)
(74) Mandataire: Desaix, Anne
(86) Numéro de dépôt international: PCT/FR1995/001133
(87) Numéro de publication internationale: WO 1996/007745

(56) Documents cités:
- JOURNAL OF BACTERIOLOGY, vol. 175, no. 16, pages 5186-5192, XP002002892 DAS GUPTA, S. ET AL.: "Cloning and assessment of Mycobacterial promoters by using a plasmid shuttle vector" cité dans la demande
- MOLECULAR MICROBIOLOGY, vol. 12, no. 3, pages 491-504, XP002002893 TIMM, J. ET AL.: "Transcription and expression analysis, using lacZ and phoA gene fusions, of Mycobacterium fortuitum beta-lactamase genes cloned from a natural isolate and a high-level beta-lactamase producer" cité dans la demande
- INFECTION AND IMMUNITY, vol. 59, no. 1, WASHINGTON US, pages 372-382, XP002002894 NAGAI, S. ET AL.: "Isolation and partial characterization of major protein antigens in the culture fluid of Mycobacterium tuberculosis" cité dans la demande
- JOURNAL OF EXPERIMENTAL MEDICINE, vol. 178, pages 197-209, XP002002895 STOVER, K. ET AL.: "Protective immunity elicited by recombinant Bacille Calmette-Guerin (BCG) expressing outer surface protein A (OspA) lipoprotein: a candidate Lyme disease vaccine" cité dans la demande
- JOURNAL OF BACTERIOLOGY, vol. 169, no. 4, pages 1663-1669, XP002002896 BOQUET, P. ET AL.: "Use of TnphoA to detect genes for exported proteins in Escherichia coli: identification of the plasmid-encoded gene for a periplasmic acid phosphatase" cité dans la demande
- SCANDINAVIAN JOURNAL OF IMMUNOLOGY, vol. 36, pages 823-831, XP002002897 ANDERSEN, P. ET AL.: "Identification of immunodominant antigens during infection with Mycobacteria tuberculosis"
- JOURNAL OF BACTERIOLOGY, vol. 176, no. 21, pages 6749-6753, XP002002898 TIMM, J. ET AL.: "Escherichia coli-Mycobacteria shuttle vectors for operon and gene fusions to lacZ: the pJEM series"
- INFECTION AND IMMUNITY 63 (7). 2581-2586, Juillet 1995, XP002002899 BIGI, F. ET AL.: "Characterization of a novel Mycobacterium bovis secreted antigen containing PGLTS repeats."
- JOURNAL OF BACTERIOLOGY, vol. 177, no. 1, Janvier 1995, pages 59-65, XP000560419 LIM, E. ET AL.: "Identification of Mycobacterium tuberculosis DNA sequences encoding exported proteins by using phoA gene fusions"

## Description

Le genre Mycobacterium inclut des pathogènes humains majeurs tels que M. leprae et M. tuberculosis, les agents responsables de la lèpre et de la tuberculose, qui restent des problèmes graves de santé publique dans le monde entier.

M. bovis et M. tuberculosis, les agents causals de la tuberculose, sont des bactéries facultatives intracellulaires. En dépit des problèmes majeurs de santé liés à ces organismes pathogènes, on sait peu de choses sur leurs protéines exportées et/ou sécrétées. Des analyses en SDS-PAGE de filtrat de culture de M. tuberculosis montrent au moins 30 protéines sécrétées (1,19,38). Certaines d'entre elles ont été caractérisées, leurs gènes clonés et séquencés (7,35,37). D'autres, bien qu'il s'agisse d'antigènes immunodominants d'importance majeure pour induire une immunité protectrice (2,21), ne sont pas totalement identifiés. En outre, il est probable que de nombreuses protéines exportées restent fixées sur la membrane cellulaire et par conséquent ne soient pas présentes dans les surnageants de culture._Il a été montré que les protéines localisées à la surface externe de diverses bactéries pathogènes, telles que l'invasine de 103 kDa de Yersina pseudotuberculosis (14) ou l'internaline de 80 kDa de Listeria monocytogenes (10) jouent un rôle important dans les interactions avec les cellules hôtes et par conséquent, dans la pathogénicité comme dans l'induction de réponses protectrices. Ainsi, une protéine liée à la membrane pourrait être importante pour l'infection à M. tuberculosis comme pour l'induction de réponse protectrice contre cette infection. Ces protéines pourraient revêtir un intérêt certain pour la préparation de vaccins.

Le BCG (Bacille de Calmette et Guérin), une souche avirulente dérivée de M. bovis, a été très utilisé comme vaccin contre la tuberculose. C'est également un vecteur très intéressant pour la construction de vaccins recombinants vivants, particulièrement en raison de son pouvoir immunogène fort. Par conséquent, l'étude de la biologie moléculaire des mycobactéries suscite actuellement beaucoup d'intérêt.

Le développement de nouveaux vaccins contre les mycobactéries pathogènes, ou l'amélioration des vaccins disponibles nécessitait la mise au point d'outils spécifiques permettant d'isoler ou d'obtenir des séquences de polypeptides immunogènes.

Les inventeurs ont défini et réalisé dans ce but de nouveaux vecteurs permettant le criblage de séquences d'ADN de mycobactéries afin d'identifier parmi ces séquences, des acides nucléiques codant pour des protéines d'intérêt.

Des vecteurs ont été définis pour évaluer l'efficacité de séquences de régulation de l'expression au sein de mycobactéries.

L'invention vise aussi de nouveaux polypeptides de mycobactéries ayant pu être isolés au moyen des vecteurs précédents et susceptibles d'entrer dans la réalisation de compositions pour la détection d'une infection par des mycobactéries, ou pour la protection contre une infection due à des mycobatéries.

La demande décrit un vecteur recombinant de criblage et/ou de clonage et/ou d'expression, caractérisé en ce qu'il se réplique chez des mycobactéries, en ce qu'il contient
- 1) un réplicon fonctionnel chez les mycobactéries ;
- 2) un marqueur de sélection ;
- 3) une cassette reporteur comprenant
   a) un site de clonage multiple (polylinker),
   b) un terminateur de transcription actif chez les mycobactéries, en amont du polylinker, et
   c) une séquence nucléotidique codante issue d'un gène codant pour un marqueur d'expression, et/ou d'exportation et/ou de sécrétion de protéine, ladite séquence nucléotidique étant dépourvue de son codon d'initiation et de ses séquences de régulation.

Le marqueur d'exportation et/ou de sécrétion est une séquence de nucléotides dont l'expression suivie de l'exportation et/ou de la sécrétion dépend des éléments de régulation qui contrôlent son expression.

Par "séquences ou éléments de régulation de l'expression", on entend une séquence promotrice de la transcription, une séquence comprenant le site de liaison au ribosome (RBS), les séquences responsables de l'exportation et/ou la sécrétion telles que la séquence dite séquence signale.

Un premier marqueur intéressant d'exportation et/ou d'expression est une séquence codante issue du gène phoA. Le cas échéant, elle est tronquée de telle façon que l'activité phosphatase alcaline est cependant susceptible d'être restaurée lorsque la séquence codante tronquée est placée sous le contrôle d'un promoteur et d'éléments de régulation appropriés.

D'autres marqueurs d'exposition et/ou d'exportation et/ou de sécrétion peuvent être utilisés. On citera à titre d'exemples une séquence du gène de la β-agarase ou de la nucléase d'un staphylocoque ou de la β-lactamase d'une mycobactérie.

Le terminateur de transcription doit être fonctionnel chez les mycobactéries. Un terminateur avantageux est à cet égard le terminateur du coliphage T4 (tT4). D'autres terminateurs appropriés pour la réalisation de l'invention peuvent être isolés en utilisant la technique présentée dans les exemples, par exemple au moyen du vecteur pJN3.

Un vecteur particulier décrit est le plasmide pJEM11 déposé à la CNCM (Collection Nationale de Cultures de Microorganismes à Paris - France) sous le n°I-1375, le 3 novembre 1993.

Pour la sélection, ou l'identification de séquences d'acides nucléiques de mycobactéries codant pour des produits susceptibles d'être incorporés dans des compositions immunogènes ou antigéniques pour la détection d'une infection par des mycobactéries, le vecteur décrit comprendra, en l'un des sites du polylinker, une séquence de nucléotides d'une mycobactérie chez laquelle on recherche la présence de séquences régulatrices associées à tout ou partie d'un gène d'intérêt permettant, lorsque le vecteur portant ces séquences (vecteur recombinant) est intégré ou se réplique dans un hôte cellulaire de type mycobactérie, d'obtenir l'exposition au niveau de la membrane ou de la paroi cellulaire de l'hôte et/ou l'exportation et/ou la sécrétion du produit d'expression de la susdite séquence de nucléotides.

La séquence en question de mycobactéries peut être toute séquence dont on cherche à détecter si elle contient des éléments de régulation de l'expression associés à tout ou partie d'un gène d'intérêt et susceptibles de permettre ou de favoriser l'exposition au niveau de la membrane cellulaire d'un hôte chez laquelle elle serait exprimée et/ou l'exportation et/ou la sécrétion d'un produit d'expression d'une séquence codante donnée et à titre d'essai du marqueur d'exportation et/ou de sécrétion.

De préférence, cette séquence est obtenue par digestion enzymatique de l'ADN génomique ou de l'ADN complémentaire d'un ARN d'une mycobactérie et de préférence d'une mycobactérie pathogène.

Selon un premier mode de réalisation du vecteur décrit, la digestion enzymatique de l'ADN génomique ou de l'ADN complémentaire est effectuée à partir de M. tuberculosis.

De préférence cet ADN est digéré avec une enzyme telle que sau3A.

D'autres enzymes de digestion telles que ScaI, ApaI, ScaII, KpnI ou encore des exonucléases ou des polymérases, peuvent naturellement être mises en oeuvre, dès lors qu'elles permettent l'obtention de fragments dont les extrémités peuvent être insérées dans l'un des sites de clonage du polylinker du vecteur de l'invention.

Le cas échéant, des digestions avec différentes enzymes seront effectuées simultanément.

Des vecteurs recombinants décrits sont choisis parmi les vecteurs recombinants suivants déposés à la CNCM le 8 Août 1994 :
pExp53 déposé à la CNCM sous le n°I-1464
pExp59 déposé à la CNCM sous le n°I-1465
pExp410 déposé à la CNCM sous le n°I-1466
pExp421 déposé à la CNCM sous le n°I-1467.

Les vecteurs de l'invention peuvent également être utilisés pour déterminer la présence de séquences d'intérêt, selon ce qui a été exposé précédemment, chez des mycobactéries telles que M. africanum, M. bovis, M. avium ou M. leprae dont on aura traité l'ADN ou l'ADNc avec des enzymes déterminées.

La demande décrit aussi un procédé de criblage de séquences de nucléotides issues de mycobactéries, pour déterminer la présence au sein de ces séquences, d'éléments - de régulation contrôlant l'expression dans un hôte cellulaire de séquences d'acide nucléique les contenant et/ou l'exposition à la surface de l'hôte cellulaire et/ou l'exportation et/ou la sécrétion des séquences polypeptidiques résultant de l'expression des susdites séquences de nucléotides, caractérisé en ce qu'il comprend les étapes suivantes :
a) la digestion des séquences d'ADN de mycobactéries par au moins une enzyme déterminée et la récupération des fragments de digestion obtenus,
b) l'insertion des fragments de digestion dans un site de clonage compatible avec l'enzyme de l'étape
a) du polylinker d'un vecteur ci-dessus,
c) si besoin, l'amplification du fragment de digestion contenu dans le vecteur, par exemple par réplication de ce dernier après insertion du vecteur ainsi modifié dans une cellule déterminée, par exemple E coli,
d) la transformation d'hôtes cellulaires par le vecteur amplifié à l'étape c), ou en l'absence d'amplification, par le vecteur de l'étape b),
e) la culture des hôtes cellulaires transformés dans un milieu permettant la mise en évidence du marqueur d'exportation et/ou de sécrétion contenu dans le vecteur,
f) la détection des hôtes cellulaires positifs (colonies positives) pour l'expression du marqueur d'exposition et/ou d'exportation et/ou de sécrétion,
g) l'isolement de l'ADN des colonies positives et l'insertion de cet ADN dans une cellule identique à celle de l'étape c),
h) la sélection des insertions contenues dans le vecteur, permettant l'obtention de clones positifs pour le marqueur d'exportation et/ou de sécrétion,
i) l'isolement et la caractérisation des fragments d'ADN de mycobactéries contenues dans ces insérats.

La mise en oeuvre de ce procédé permet la construction de banques d'ADN comportant des séquences susceptibles d'être exportées et/ou sécrétées, lorsqu'elles sont produites au sein de mycobactéries recombinantes.

L'étape i) du procédé peut comprendre une étape de séquençage des insertions sélectionnées.

De préférence, le vecteur utilisé est le plasmide pJEM11 (CNCM I-1375) et la digestion est effectuée au moyen de l'enzyme sau3A.

Le procédé de criblage est par exemple caractérisé en ce que les séquences de mycobactéries sont issues d'une mycobactérie pathogène, par exemple de M. tuberculosis, M. bovis, M. avium, M. africanum ou M. leprae.

La demande décrit aussi les séquences nucléotidiques de mycobactéries sélectionnées après la réalisation du procédé ci-dessus décrit.

Des séquences sélectionnées sont par exemple les fragments d'ADN de mycobactéries contenus dans les vecteurs pIPX412 (CNCM I-1463 déposé le 8/08/94), pExp53, pExp59, pExp410 ou pExp421.

Lorsque la séquence codante issue du gène marqueur d'exportation et/ou de sécrétion est une séquence issue du gène phoA, l'exportation et/ou la sécrétion du produit du gène phoA, le cas échéant tronqué, n'est obtenue que lorsque cette séquence est insérée en phase avec la séquence placée en amont, qui contient les éléments contrôlant l'expression et/ou l'exportation et/ou de sécrétion issus de séquence de mycobactéries.

La demande décrit aussi des mycobactéries recombinantes contenant un vecteur recombinant décrit précédemment. Une mycobactérie préférée est une mycobactérie du type M. smegmatis.

M. smegmatis permet avantageusement de tester l'efficacité de séquences de mycobactéries, pour le contrôle de l'expression et/ou de l'exportation et/ou de la sécrétion d'une séquence donnée, par exemple d'une séquence codant pour un marqueur tel que la phosphatase alcaline.

Une autre mycobactérie intéressante est une mycobactérie du type M. bovis, par exemple la souche BCG utilisée actuellement pour la vaccination contre la tuberculose.

La demande décrit par ailleurs une mycobactérie recombinante, caractérisée en ce qu'elle contient un vecteur recombinant ci-dessus défini.

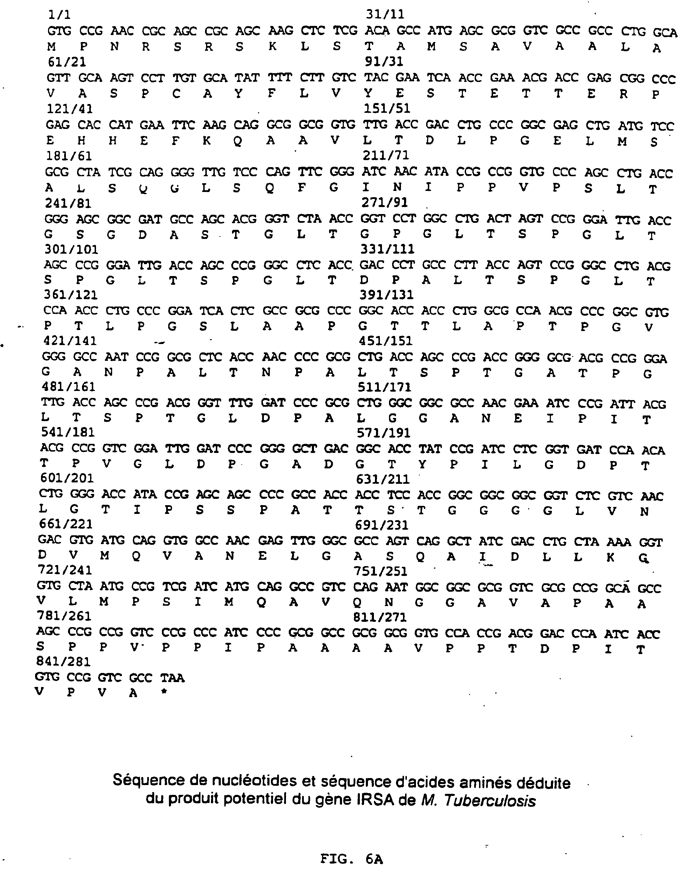

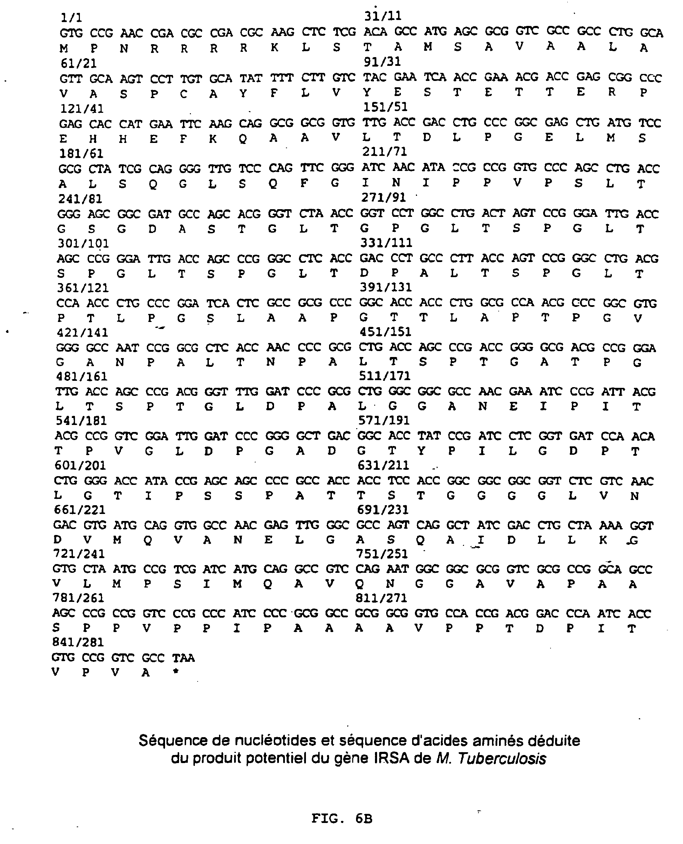

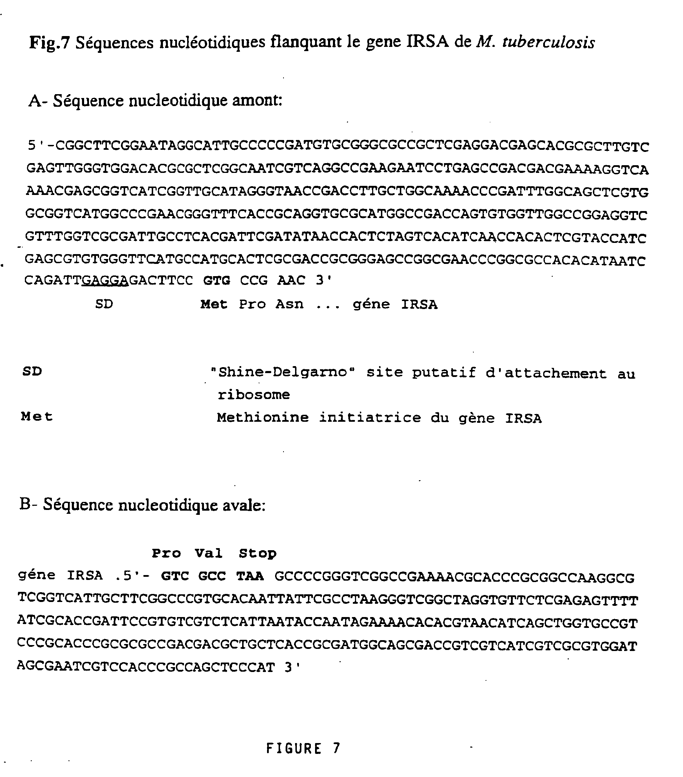

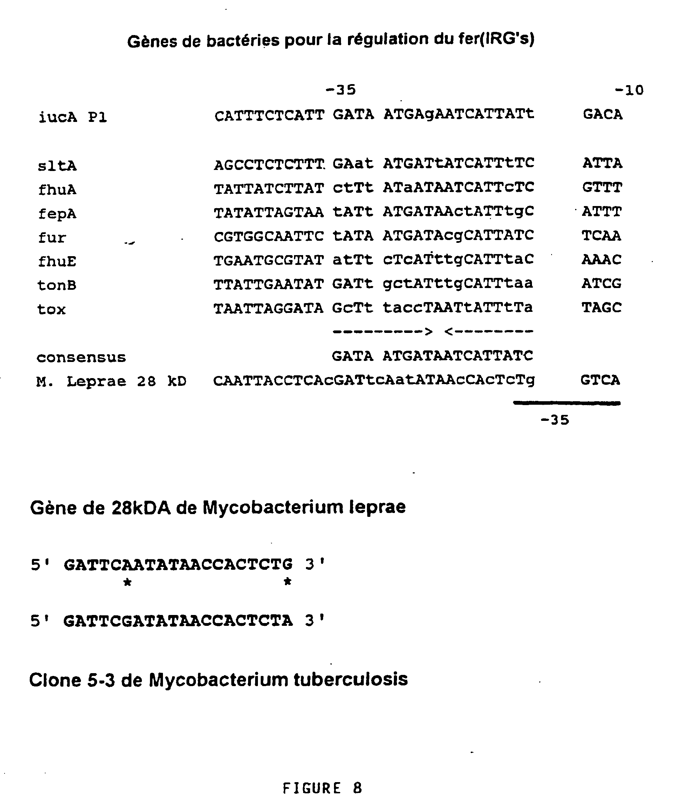

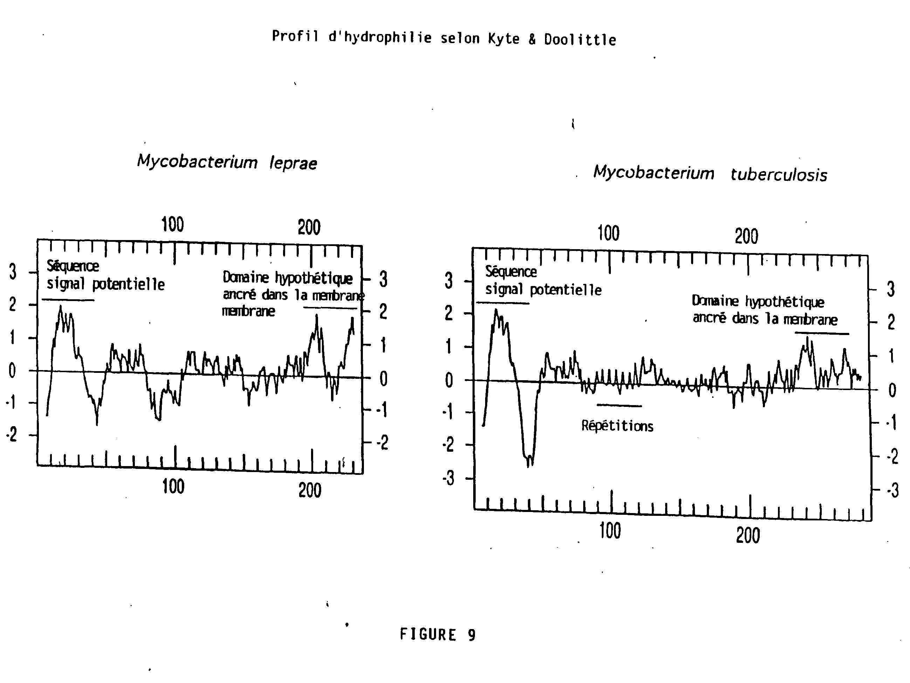

L'invention a pour objet une séquence de nucléotides issue d'un gène codant pour une protéine exportée de M. tuberculosis, caractérisée en ce qu'elle est choisie parmi les séquences suivantes :
- une séquence IA répondant à l'enchaînement de nucléotides décrit à la figure 6A, ou une séquence IB répondant à l'enchaînement de nucléotides décrit à la figure 6B, ou une séquence codant pour une protéine exportée de M. tuberculosis et hybridant dans des conditions de stringence élevée avec ces enchaînements,
- une séquence comprenant l'enchaînement de nucléotides IA ou IB et codant pour une protéine P28 de M. tuberculosis ayant un poids moléculaire théorique d'environ 28kDa et un poids moléculaire observé de 36kDa, déterminé par éléctrophorèse en gel d'acrylamide dénaturant (PAGE-SDS)
- une séquence comprise dans la séquence IA ou IB et codant pour un polypeptide reconnu par des anticorps dirigés contre la protéine P28 de M. tuberculosis, répondant à l'enchaînement d'acides aminés VIII A-représenté à la figure 6A ou à l'enchainement d'acides aminés VIIIB représenté à la figure 6B,
- une séquence IV comprenant les séquences régulatrices du gène comprenant la séquence codante IA ou IB, lesdites séquences étant contenues dans les séquences nucléotidiques amont et aval représentées à la figure 7A et à la figure 7B,
- une séquence V répondant à l'enchaînement compris entre les nucléotides 1 et 72 de la séquence IA ou IB et correspondant à la séquence signal,
- une séquence VI répondant à l'enchaînement compris entre les nucléotides 62 à 687 de la séquence IA ou IB,
- une séquence VII répondant à l'enchaînement compris entre les nucléotides 688 et 855 de la séquence IA ou IB.

Entre également dans le cadre de l'invention, un polypeptide de M. tuberculosis, caractérisé en ce qu'il répond à l'enchaînement d'acides aminés VIIIA ou à l'enchaînement VIIIB représentés à la figure 6A et 6B respectivement ou en ce qu-' il comprend l'un de ces enchaînements.

Un polypeptide préféré est caractérisé en ce qu'il a un poids moléculaire théorique d'environ 28kDa, déterminé selon la technique décrite dans les exemples.

La protéine p28 de M. tuberculosis a été caractérisée par sa capacité à être exportée et donc potentiellement localisée à travers la membrane bactérienne plasmatique ou la paroi cellulaire. De plus, comme le montrent les séquences présentées à la figure 6, certains motifs peptidiques de la séquence sont répétés. Pour ces raisons, la protéine p28 de M. tuberculosis est maintenant le plus souvent désignée comme protéine ERP et le gène contenant la séquence codante de cette protéine est appelé soit gène irsa, soit gène erp.

Le poids moléculaire théorique de la protéine ERP, évalué à 28 kDa, correspond à un poids moléculaire observé expérimentalement d'environ 36 kDa (migration électrophonétique en gel de polyacrylamide dénaturant (PAGE-DOS)).

Un autre polypeptide intéressant dans le cadre de l'invention comprend une partie de l'enchaînement VIII ou VIIIB d'acides aminés précédemment décrit et réagit immunologiquement avec des anticorps dirigés contre la protéine p28 de M. tuberculosis.

Des séquences d'acides aminés particulièrement intéressantes dans le cadre de l'invention sont les séquences comprenant l'un des enchaînements suivants en une ou plusieurs copies :
PGLTS,PGLTD,PGLTP,PALTN,PALTS,PALGG,PTGAT,PTGLD,PVGLD.

D'autres séquences intéressantes sont par exemple la séquence signal comprise entre les positions de nucléotides 1 et 72 des séquence de la figure 6A ou 6B ou encore la séquence comprise entre les nucléotides 688 et 855 susceptible de se comporter comme une séquence transmembranaire.

Ces séquences polypeptidiques peuvent être exprimées sous la forme de polypeptides recombinants. Dans ces polypeptides recombinants, elles peuvent être remplacées en partie notamment en ce qui concerne les séquences de 5 acides aminés précédemment décrites, par des séquences d'intérêt provenant de mycobactéries ou d'autres organismes pathogènes, ce remplacement pouvant conduire à inclure, au sein des polypeptides recombinants, des épitopes ou des déterminants antigéniques d'un organisme pathogène ou d'une protéine d'intérêt contre lequel (laquelle) on chercherait à obtenir des anticorps.

Ainsi, les polypeptides de l'invention, tout en présentant éventuellement eux-mêmes des propriétés antigéniques, voire immunogènes, peuvent être utilisés comme molécules porteuses intéressantes pour préparer, le cas échéant, des vaccins ayant des propriétés variées.

L'invention a également pour objet des anticorps monoclonaux ou des sérums polyclonaux dirigés contre un polypeptide tel que défini précédemment.

S'agissant des anticorps monoclonaux, ils sont de préférence dirigés spécifiquement contre un polypeptide de l'invention et ne reconnaissent par exemple pas la protéine p28 de M. leprae.

L'invention a aussi pour objet une composition pour la détection in vitro d'une infection par M. tuberculosis, caractérisée en ce qu'elle comprend un polypeptide ci-dessus défini, capable de réagir immunologiquement avec des anticorps formés chez un patient infecté par M. tuberculosis.

Une autre composition pour la détection in vitro d'une infection par M. tuberculosis est caractérisée par une séquence de nucléotides contenant au moins 9 nucléotides, issue d'une séquence ci-dessus définie, ou une séquence de nucléotides contenant au moins 9 nucléotides et hybridant dans des conditions stringentes avec l'ADN de M. tuberculosis et n'hybridant pas dans les mêmes conditions avec l'ADN de M. leprae, cette séquence étant une séquence d'ADN ou d'ARN, le cas échéant marquée.

L'invention a aussi pour objet un hôte cellulaire procaryote ou eucaryote caractérisé en ce qu'il est transformé par une séquence de nucléotides telle que décrite dans les pages précédentes, dans des conditions permettant l'expression de cette séquence et/ou son exposition au niveau de la membrane de l'hôte cellulaire et/ou son exportation et/ou sa sécrétion a partir de la susdite membrane.

A titre préféré, les hôtes cellulaires sont des mycobactéries telles que M. smegmatis ou M. bovis BCG.

D'autres hôtes cellulaires sont par exemple E. coli, des cellules CHO, BHK, Spf9/Baculovirus, des levures telles que Saccharomyces cerevisiae, le virus de la vaccine.

L'invention a aussi pour objet une composition, immunogène comprenant un polypeptide tel que présenté plus haut ou un hôte cellulaire tel que défini ci-dessus.

La demande décrit par ailleurs un vecteur pour le criblage et/ou le clonage et/ou pour l'expression de séquences de nucléotides fonctionnelles dans des mycobactéries, dérivé d'un vecteur précédemment décrit et
caractérisé en ce que la séquence codante issue d'un gène codant pour un marqueur d'exportation et/ou de sécrétion est remplacée par un gène reporteur ou une séquence reporteur.

Le gène ou la séquence reporteur peut être dépourvu de ses séquences régulatrices, en particulier de ses séquences de liaison au ribosome et/ou de ses séquences permettant l'exportation et/ou la sécrétion du marqueur produit lorsque le vecteur est incorporé à un hôte cellulaire recombinant.

gène ou la séquence reporteur contient la séquence codant du gène lacZ ou une partie de cette séquence suffisante pour que le polypeptide présente une activité β-galactosidase.

Un vecteur particulier est caractérisé en ce qu'il comprend en l'un des sites de clonage du polylinker, un enchaînement de nucléotides comprenant un promoteur et le cas échéant des séquences de régulation, par exemple pour l'ancrage à la surface, l'exportation voire la sécrétion d'un polypeptide qui serait produit sous le contrôle du promoteur, dont on souhaite évaluer la capacité à promouvoir ou réguler l'expression d'une séquence nucléotidique reporteur chez des mycobactéries.

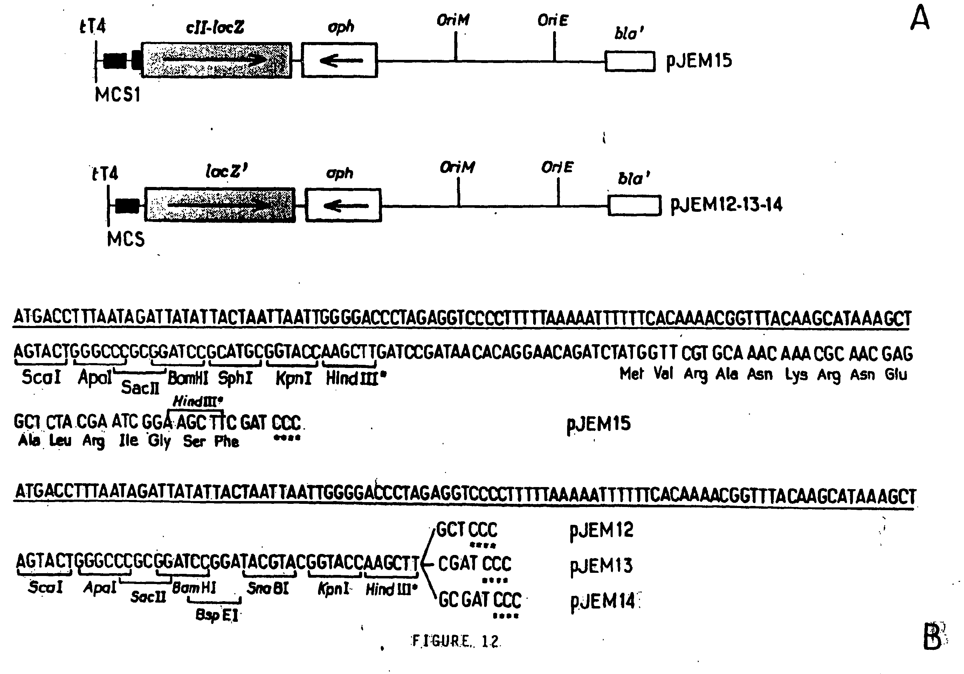

Des vecteurs particuliers sont des plasmides choisis parmi les plasmides pJEM12, pJEM13, pJEM14 ou pJEM15 tels que représentés à la figure 12.

Un tel vecteur peut être utilisé pour évaluer l'intérêt de séquences de régulation d'expression ou de promoteurs, par exemple les séquences de promoteurs pAN, pblaF*, psul3, pgroES/EL1.

La demande décrit également un procédé pour déterminer l'activité d'une séquence contenant en l'un des sites de clonage du polylinker, un enchaînement de nucléotides comprenant un promoteur et le cas échéant des séquences de régulation, par exemple pour l'exposition, l'exportation voire la sécrétion d'un polypeptide qui serait produit sous le contrôle du promoteur dans des mycobactéries, caractérisé en ce qu'il comprend les étapes de :
- transformation d'une souche de mycobactérie, par exemple M. smegmatis ou M. tuberculosis avec un vecteur ci-dessus décrit,
- détection de l'activité normalement associée à là présence du gène reporteur ou de la séquence reporteur.
   D'autres caractéristiques et avantages de l'invention apparaissent à la lecture des exemples qui suivent ainsi que dans les figures.

### LEGENDE DES FIGURES

### Fig. 1

### Construction de pJEM11.

Voir Matériel et Méthodes. pJEM11 a des origines de réplication (ori) de E. coli et des mycobactéries. Il s'agit donc d'un plasmide navette. Le marqueur de sélection est le gène de résistance à la kanamycine (Km). Le gène PhoA tronqué de pPH07 (22) est dépourvu de promoteur, de codon de départ et de séquence signal, ainsi l'expression et l'exportation de PhoA dépendent de la fusion traductionnelle avec les extrémités amino-terminales d'autres protéines. Le terminateur transcriptionnel (T) de la cassette omega évite la transcription par "read-through" à partir de séquences plasmidiques.

### Fig. 2

### Construction des plasmides pLA71, pLA72 et pLA73.

L'insertion dans le site Bam H1 de pJEM11 de fragments BlaF* (34) de 3 longueurs différentes conduit à l'expression de protéines de fusion avec l'activité phoA. Des dosages colorimétriques ont été effectués selon la technique de Brockman et Heppel (8), avec le p-nitrophényl-phosphate comme substrat. Les teneurs en protéines ont été mesurées à l'aide du dosage Bio-Rad. Les unités arbitraires de phosphatase alcaline (aU) ont été calculées comme décrit dans Matériel et Méthodes.

### Fig. 3

### Analyses de western-blot de protéines de fusion PhoA.

Des souches de M. smegmatis transformées ont été mises en culture en milieu Beck contenant de la kanamycine (20 µg/ml). Des extraits totaux de bactéries soniquées ont été solubilisés par du SDS, résolus par SDS-PAGE et soumis à immunoempreinte. La préparation du sérum de lapin anti-PhoA a été décrite précédemment (34). Des anticorps de lapin couplés à PhoA (Promega) et, comme substrat, un mélange de X-P et de nitrobleutétrazolium (BCIP-NBT, Promega) ont été utilisés pour révéler les fusions PhoA. Colonne 1: PhoA bactérienne purifiée, M. smegmatis transformée par des plasmides pJEM11: colonne 2, pLA71: colonne 3, pLA72: colonne 4, pLA73: colonne 5, pExp410: colonne 6, pExp53: colonne 7, pExp59: colonne 8, pExp421: colonne 9

### Fig. 4

### Séquences nucléotidiques et d'acides aminés déduites de segments d'insérats sélectionnés des plasmides pExp410, pExp53, pExp59 et pExp421.

Les clones de M. smegmatis avec l'activité de la phosphatase alcaline ont été sélectionnés sur des boites X-P/kanamycine. Leurs plasmides ont été amplifiés chez E. coli XL-1 B, et la séquence nucléotidique des insérats déterminée comme décrit dans Matériel et Méthodes. A: pExp410 inclut une partie de la lipoprotéine de 19 kDa. Le cadre de lecture est maintenu à la jonction avec phoA (Bam H1/Sau 3A). B: pExp53 inclut une partie d'un gène présentant des similitudes avec l'antigène de 28 kDa de M. leprae. Les acides aminés divergents sont en caractères gras. Le codon de début de traduction est GTG. Les sites de clivage putatifs par signal peptidase sont indiqués par des flèches. C: pExp59 code pour une séquence signal caractéristique. Un site de liaison aux ribosomes putatif (RBS) est souligné. Le site putatif de clivage par la signal peptidase est indiqué par une flèche. D: pExp421 code pour des motifs d'acides aminés conservés avec des protéines de la famille des stéaroyle- protéine-porteuse-d'acyle (ACP) désaturases. R. comm: R. communis (ricin).

### Fig. 5

### Le gène similaire au gène de l'antigène de 28 kDa de M. leprae est présent en une seule copie dans le génome de M. tuberculosis.

L'ADN génomique de M. tuberculosis a été extrait en suivant des protocoles standard (27), digéré par des endonucléases Pst I, Sma I, Bst EII, Sph I, Bam HI et soumis à une migration sur un gel d'agarose à 1 %. L'hybridation de Southern-blot a été effectuée selon des protocoles standard (27). La sonde marquée au 32P était un fragment de PCR de 180 pb de l'insérat de pExp53.

### Fig 6

Séquence de nucléotides (IA et IB) et d'acides aminés (VIIIA et VIIIB) du produit du gène IRSA codant pour la protéine P28 de M. tuberculosis (deux variantes sont présentées). Ce gène est maintenant désigné par l'abréviation "erp" correspondant à l'expression "exported repetitive protein".

### Fig. 7

**Séquences de nucléotides préliminaires flanquant le gène IRSA de M. tuberculosis.**

### Fig. 8

**Gènes de bactéries pour la régulation du fer (IRG's).**

### Fig. 9

**Profil d'hydrophilie DES PROT2INES P28 de M. leprae et M. tuberculosis**

### Fig. 10

A) Alignement des séquences de nucléotides du gène codant pour les protéines p28 de M. tuberculosis et de M. leprae.
B) Alignement des séquences d'acides aminés des protéines p28 de M. tuberculosis et de M. leprae.

### Fig. 11

### Construction des plasmides pJN3 et pJN11.

Seuls les sites de restriction et éléments génétiques pertinents sont montrés. Les plasmides pRR3 et pJN1 ont été décrits dans l'art antérieur (60) (58). La cassette omega a été obtenue par digestion par SmaI de pHP45X (59), suivie de la purification d'un fragment de 2 kb d'un gel d'agarose en utilisant le kit Geneclean (Bio 101 Inc.). Les techniques standard d'ADN recombinant ont été utilisées conformément à la description donnée dans l'état de la technique (61). Dans pJN3 et pJN11, le gène de la β-lactamase (bla) a été interrompu. oriE et oriM désignent les origines de réplication de pUC (E. coli) et de pAL5000 (mycobactéries), respectivement.

### Fig. 12.

### structure des plasmides de la série pJEM.

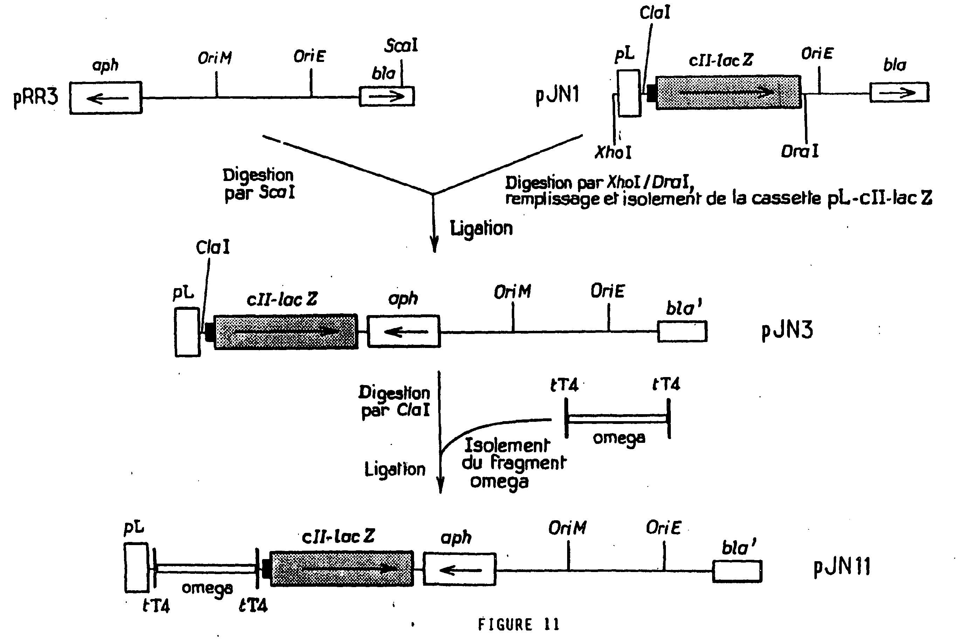

(A) Dans la représentation schématique des plasmides, seuls les éléments génétiques pertinents sont indiqués. pJEM15 a résulté du clonage, dans le site ScaI de pRR3, i) d'un fragment obtenu par amplification par PCR (en utilisant OJN1:5¹-AAGCTTCCGATTCGTAGAGCC-3'et OJN2:5'-GGGCTCGAGCTGCAG TGGATGACCTTTTGA-3' comme amorces; et pJN11 comme matrice) et contenant tT4 et l'extrémité N-terminale de cII; ii) des oligonucléotides de synthèse correspondant à MCS1; et iii) le fragment lacZ' de HindIII-DraI de pNM480. pJEM12-13-14 ont été obtenus par clonage du fragment amplifié par PCR décrit ci-dessus dans le site Scal de pRR3. Les oligonucléotides de synthèse correspondant à MCS2 ont été ensuite insérés. Enfin, chacune des trois formes de la série pNM480 a été introduite dans le site HindIII dans MCS2. (B) Séquences nucléotidiques des régions entre l'amorce OJN1 et le 8ème codon de lacZ' (marqué ****). Ces séquences ont été vérifiées expérimentalement. La région tT4 est soulignée et le RBS de synthèse est en caractères gras. La séquence d'acides aminés de l'extrémité N-terminale de cII est donnée en-dessous de la séquence d'ADN. Les sites HindIII sont marqués par un astérisque car ils ne sont pas uniques. Pour des descriptions complémentaires, voir la légende de la fig.11.

### EXEMPLES

### I) Identification de gènes codant our des protéines exportées de M. tuberculosis.

Les résultats rapportés ici décrivent la définition pour des mycobactéries d'une méthode génétique d'identification de protéines exportées. Cette méthodologie est basée sur la fusion traductionnelle avec la phosphatase alcaline bactérienne (Phoa). De telles protéines de fusion doivent être exportées pour avoir l'activité PhoA (6,13,16). Un gène PhoA a été utilisé après délétion de la région promoteur, du site de liaison aux ribosomes et de l'ensemble de la région codant pour la séquence signale dont le codon de début de traduction a été utilisé. Ainsi, l'activité phosphatase alcaline est dépendante de la fusion traductionnelle réalisée dans la bonne phase de lecture avec une partie d'une protéine exportée. La construction d'un vecteur plasmidique phoA pour les mycobactéries est décrite en premier lieu puis on a montré que l'introduction dans ce vecteur du gène de la β-lactamase exportée de M. fortuitum (blaF*)(34) conduit à la production chez M. smegmatis de protéines de fusion présentant l'activité enzymatique PhoA. Une banque de séquences de fusion entre l'ADN génomique de M. tuberculosis et le gène phoA a ensuite été construite. Douze clones indépendants qui exportaient de protéines de fusion ont été isolés. Parmi eux, on a pu identifier la lipoprotéine exportée de 19 kDa déjà décrite chez M. tuberculosis, une nouvelle séquence de M. tuberculosis présentant des similitudes avec la protéine de 28 kDa de M. leprae, une protéine comportant des résidus d'acides aminés conservés avec des stéaroyle- protéine-porteuse-d'acyle (ACP) désaturases, et d'autres séquences nouvelles.

### MATERIEL ET METHODES

### Souches bactériennes, plasmides, et conditions de culture

Les souches bactériennes et les plasmides utilisés dans cette étude sont présentés au tableau 1. La croissance de souches de E. coli et de M. smegmatis, l'électroporation, le criblage sur gélose contenant 20 µg/ml de kanamycine et 20 µg/ml de phosphate de 5-bromo-4-chloro-3-indolyle (X-P) ont été réalisés comme décrit précédemment (14)

M. tuberculosis, isolat d'un patient (souche 103), a été cultivé sur milieu solide de Lowënstein-Jensen.

### Manipulation et séquençage d'ADN

La manipulation d'ADN et les analyses de Southern-blot ont été effectuées à l'aide de techniques standard (27). Pour les déterminations des séquences, les oligonucléotides (5-GGCCCGACGAGTCCCGC-3' et 5'-TTGGGGACCCTAGAGGT-3') ont été mis au point en vue du séquençage à travers les jonctions de fusion des insérats de M. tuberculosis dans pJEM11 (voir ci-dessous). Les séquences d'ADN plasmidique à double brin ont été déterminées par la méthode de terminaison de chaînes par des dideoxy (28) en utilisant le kit de séquençage T7 (Pharmacia) selon les instructions du fabricant, ou avec le kit de séquençage Cycle Terminateur Taq Dyc Désoxy (Applied Biosystems), sur un système de PCR GeneAmp 9600 (Perkin Elmer), et passées sur un système d'analyse d'ADN - Modèle 373 (Applied Biosystems).

### Analyses des banques de données

Les séquences nucléotidiques ont été comparées à celles des banques de données d'EMBL et de GenBank en utilisant l'algorithme FASTA (23) et les séquences de protéines dérivées ont été analysées pour déterminer une similitude éventuelle avec les séquences contenues dans les banques de données de protéines PIR et SwissProt en utilisant l'algorithme BLAST (1).

### Constructions des plasmides

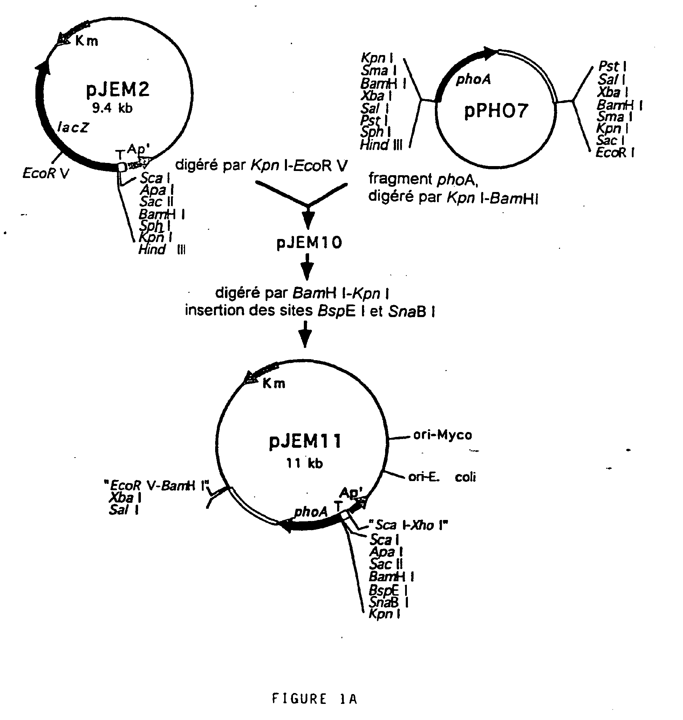

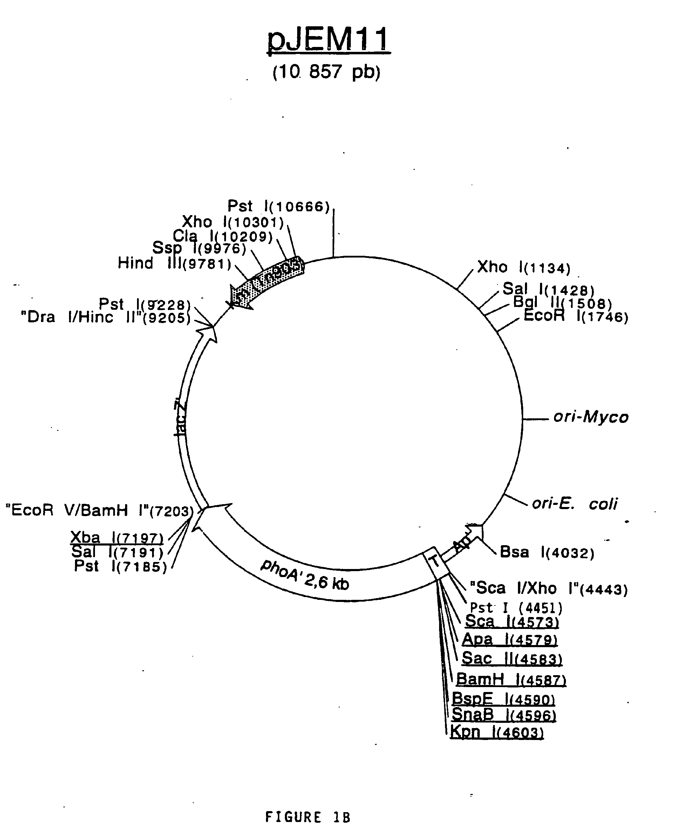

pJEM11: La construction de pJEM11 est résumée sur la fig. 1. Brièvement, pJEM2 a été construit à partir du plasmide navette pRR3 de E. coli-mycobactéries (26), par insertion du fragment lacZ tronqué de pNM480 (18), d'un multisite de clonage ou poly-linker (MCS), et du terminateur transcriptionnel de la cassette omega (24). Le fragment N-terminal EcoRV-KpnI de lacZ est remplacé par le fragment phoA tronqué de pPHO7 (11), sans codon de départ ou de séquence signal pour donner pJEM10. Enfin, un codon de départ potentiel dans le MCS a été éliminé pour donner pJEM11.

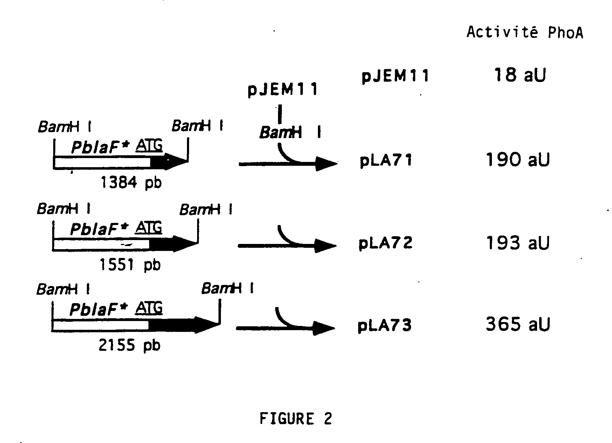

pLA71, pLA72, et pLA73 : Des fragments de longueur différente de blaF*(34) obtenus par amplification par PCR ont été insérés au site Bam H1 de pJEM11 pour donner pLA71, pLA72, et pLA73 (fig. 2). Les oligonucléotides (Genset, Paris) utilisés pour l'amplification par PCR étaient vers l'Avant 5'-CGGGATCCTGCTCGGCGGACTCCGGG-3' et vers l'Arrière 5'-CGGGATCCGGTCATCGATCGGTGCCGCGAA-3',5'-CGGGATCCCGCCGTGCTCGG CCATCTGCAG-3'et 5'-CGGGATCCAGAGTAAG GACGGCAGCACCAG-3', pour pLA71, pLA72 et pLA73 respectivement. Les amplifications par PCR ont été effectuées dans un Cycleur Thermique à ADN (Perkin Elmer), en utilisant la Taq polymérase (Cetus), selon les recommandations du fabricant.

### Construction des banques génomiques de M. tuberculosis

De l'ADN génomique de M. tuberculosis a été extrait selon des protocoles standard (27). Cet ADN a été partiellement digéré par Sau3A (avec 1U par 2 µg) à 37°C pendant 2min 30sec. La digestion a été arrêtée par l'addition de phénol. On a alors fait migrer cet ADN sur de l'agarose à bas point de fusion (Gibco, BRL). La fraction contenant les fragments ayant de 400 à 2000 pb a été extraite par de l'agarase (GELase, Epicentre Technologies) et ligaturée au site Bam HI compatible de pJEM11 avec la T4 DNA ligase (Boehringer Mannheim), à 16°C pendant une nuit.

### Dosage de la phosphatase alcaline

Pour les dosages de la phosphatase alcaline, M. smegmatis a été cultivé dans du bouillon L additionné de tylaxopol à 0,05 % (Sigma) à 37° pendant 48 h. L'activité de la phosphatase alcaline a été dosée par la méthode de Brockman et Heppel (8), dans des extraits soniqués comme décrit précédemment (34), en utilisant le phosphate de p-nitrophényle comme substrat de la réaction. Les teneurs en protéines ont été mesurées à l'aide du dosage Bio-Rad (Bio-Rad). L'activité de la phosphatase alcaline est exprimée en Unités arbitraires (aU)=DO₄₂₀x105x1g de protéine⁻¹xmin⁻¹.

### Préparations d'anticorps, électrophorèse sur gel de SDS-polyacrylamide et immunoempreintes

La préparation d'un sérum de lapin anti-PhoA a été décrite précédemment (34). Des extraits cellulaires de M. smegmatis ont été préparés par sonication, la SDS-PAGE et l'immunoempreinte ont été réalisées comme décrit précédemment (36).

### RESULTATS

### Construction d'un vecteur plasmidique navette (pJEH11) pour la production de protéines de fusion avec PhoA chez M. smegmatis

pJEM11 possède un gène phoA tronqué de E. coli sans codon d'initiation ou éléments de régulation quelconques (fig. 1). Le site multiple de clonage permet l'insertion de fragments provenant de gènes codant pour des protéines exportées putatives en même temps que leurs éléments de régulation. Ainsi, des protéines de fusion ont pu être produites, elles exprimaient l'activité de la phosphatase alcaline lorsque la fusion était exportée. pJEM11 est un plasmide navette E. coli/mycobacteries qui inclut le gène de résistance à l'antibiotique kanamycine de tn903 comme marqueur de sélection.

### L'insertion d'éléments génétiques responsables de l'expression et de l'exportation de β-lactamase chez pJEM11 conduisent à la production de protéines de fusion PhoA enzymatiquement actives chez M. smegmatis

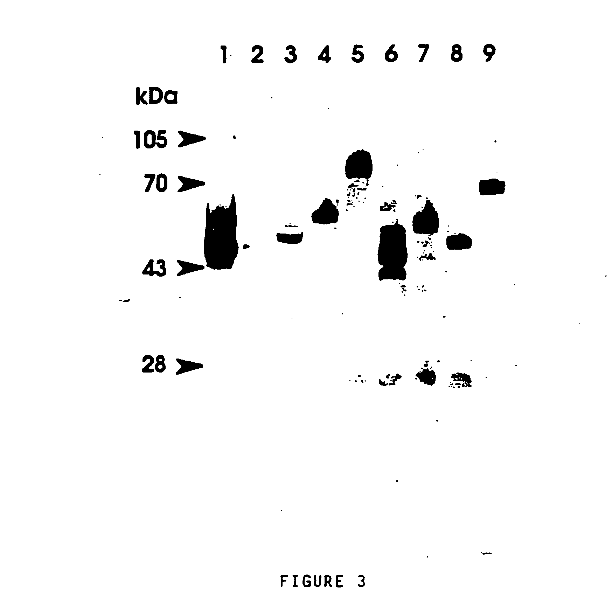

Les trois plasmides ont été construits par insertion de fragments de différente longueur provenant du gène de la β-lactamase de la souche surproductrice M. fortuitum D316 (blaF*) (34) au site Bam HI de pJEM11 (fig. 2). Dans pLA71, le fragment de 1384 pb inclut le promoteur, le segment codant pour les 32 acides aminés de la séquence signal, et les 5 premiers acides aminés de la protéine mature (il n'y a pas de séquence Shine-Dalgarno pour la fixation ribosomique dans la séquence d'origine de blaF*). pLA72 porte un fragment de 1550 pb incluant les éléments codant pour la séquence signal et les 61 premiers acides aminés de la protéine mature. Dans pLA73, le fragment de 2155 pb contient la blaF* entière. Ces plasmides ont été utilisés pour transformer M. smegmatis et les transformants ont été criblés pour les fusions PhoA enzymatiquement actives par étalement sur milieux gélosés contenant de la kanamycine et du X-P. Le X-P est soluble et incolore, mais après clivage du phosphate par la phosphatase alcaline, un précipité bleu est produit. Ainsi, des clones produisant la phosphatase alcaline pouvaient être facilement identifiés par leur coloration bleue. L'expression de pLA71, 72 et 73 chez M. smegmatis, conduit à des colonies bleues, alors que des colonies avec pJEM11 sont restées blanches. Des analyses de Western-blot ont montré la production de protéines de fusion phoA avec un poids moléculaire apparent d'environ 47,5 kDa, 54 kDa, et 76 kDa, pour pLA71, pLA72 et pLA73 respectivement (fig. 3, colonne 3,4,5). Ces poids moléculaires sont en accord avec la longueur de la protéine mature fusionnée à la phosphatase alcaline (P.M. apparent de 46 kDa, fig. 3, colonne 1). Dans pJEM11, il n'y a pas d'expression de PhoA, comme prévu (fig. 3, colonne 2). Le dosage de l'activité de la phosphatase alcaline (voir la fig. 2) de ces bactéries confirme l'expression d'une activité enzymatique avec les 3 constructions pLA. Toutefois, M. smegmatis avec pLA73 exprime environ deux fois plus d'activité comparée à pLA73 et 72. Dans des expériences distinctes, nous avons confirmé que la production intracellulaire de phoA sous le contrôle d'un promoteur mycobactérien, sans fusion avec une protéine exportée, n'était pas associée à l'expression de l'activité de la phosphatase alcaline. L'ensemble de ces résultats indique que dans ce système, l'activité de la phosphatase alcaline dépend de la fusion traductionnelle et de l'exportation proprement dite du produit. Par conséquent, pJEM11 convient à l'identification génétique des protéines exportées par les mycobactéries.

### Construction chez M. smegmatis d'une banque de fusions phoA avec des fragments d'ADN génomique de M. tuberculosis

L'ADN génomique d'un isolat clinique de M. tuberculosis a été purifié et partiellement digéré par Sau3A. La fraction de 400/2000 pb a été insérée au site Bam HI compatible de pJEM11. Les produits de ligation ont été transférés dans E. coli XL-1 bleu par électroporation pour obtenir une étape- d'amplification. Environ 2500 clones contenant des plasmides avec des insertions ont poussé sur un milieu gélosé contenant de la kanamycine. Les plasmides purifiés à partir des transformants ont été réunis et transférés par électroporation dans M. smegmatis MC²155. Les bactéries transformées ont été étalées sur gélose L-kanamycine-X-P. Environ 14000 clones ont été obtenus. Après 4 jours d'incubation, les premières colonies bleues donc PhoA⁺ ont été observées. Chaque jour, les boites ont été vérifiées, et de nouvelles colonies PhoA+ ont été isolées. Les colonies clonées ont été lysées, et leur ADN introduit par électroporation dans E. coli XL-1 bleu, pour les préparations de plasmides. En tout, 12 inserts différents permettant l'expression de phoA ont été isolés et séquences. Trois séquences présentaient des similitudes avec des séquences connues.

### Fusion de PhoA avec le gène de la lipoprotéine de 19 kDa de M. tuberculosis

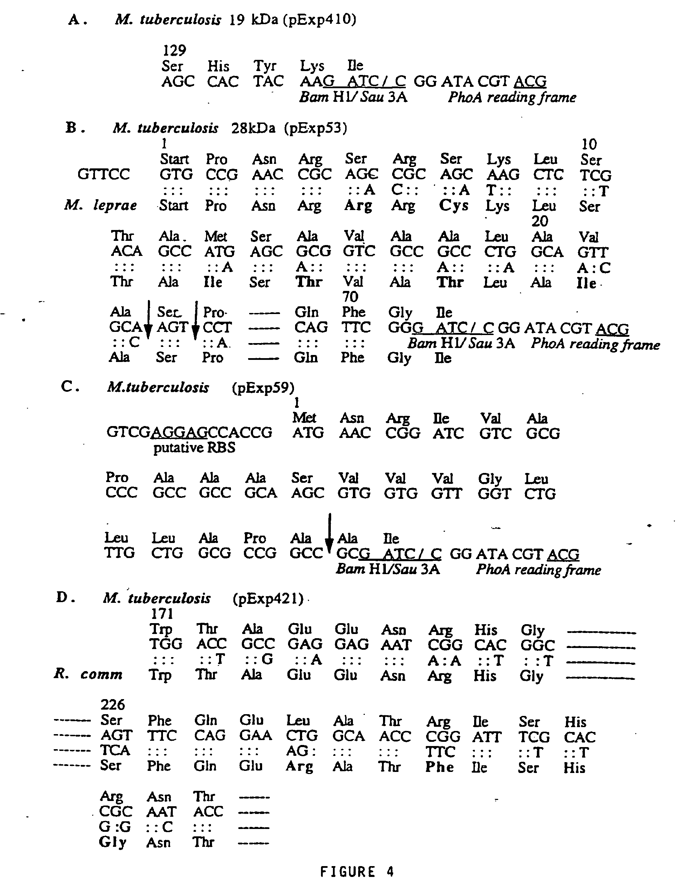

L'un des plasmides (pExp410) possède une insertion correspondant à une partie du gène de la protéine de 19 kDa déjà connue. Ce gène code pour une lipoprotéine exportée (5,31). La fig. 4 A montre la séquence d'ADN correspondant à la fusion entre ce gène et phoA. Comme prévu, la même phase de lecture est maintenue entre les deux protéines. Le poids moléculaire attendu de la protéine de fusion, selon la séquence serait près de 57 kDa. Toutefois, le vrai poids moléculaire observé par analyse de Western-blot est identique à la protéine PhoA purifiée (fig. 3, colonne 1 et 6), ce qui suggère que la protéine de fusion est clivée près de la jonction PhoA.

### Fusion avec une séquence similaire au gène de la protéine de 28 kDa de M. leprae.

La protéine de 28 kDa de M. leprae est un antigène majeur qui est très souvent reconnu par les sérums de patients atteints de la forme lépromateuse de la lèpre (9). Dans la banque d'insertion de M. tuberculosis préparée, une séquence portée par un vecteur recombinant (pExP53) présentant une similitude de 77 % avec la séquence nucléotidique de ce gène et de 68 % pour la séquence d'acides aminés déduite (fig. 4 B) a été identifiée. Dans l'analyse par Western-blot, le poids moléculaire de la protéine de fusion est d'environ 52 kDa (fig. 3, colonne 7), ce qui prévoit environ 45 acides aminés de la protéine mycobactérienne dans la protéine de fusion, après clivage du peptide signal. Ceci est conforme à la longueur du fragment du gène de M. tuberculosis fusionné avec phoA (fig. 4 B).

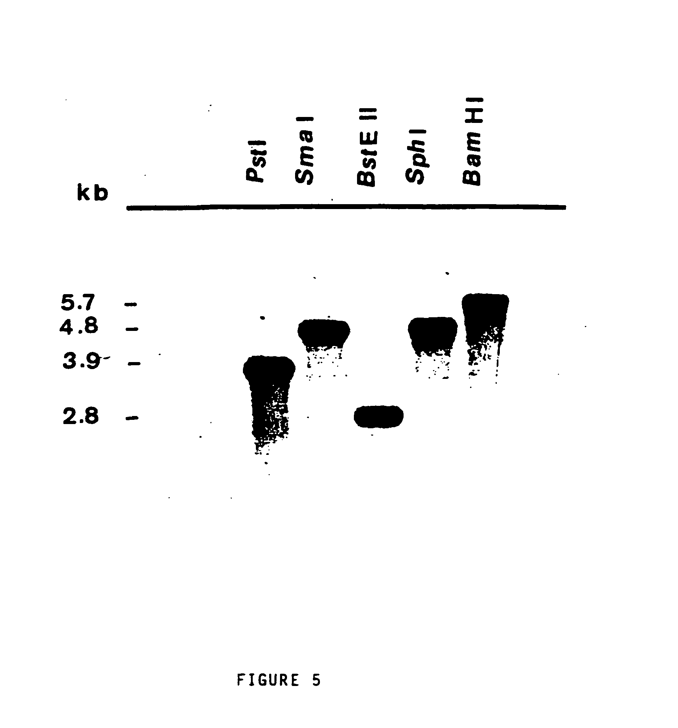

Des analyses de Southern-blot de l'ADN génomique de M. tuberculosis ont été réalisées. Il a été montré qu'un fragment de 180 pb de l'insertion de 2 kb du plasmide pExp53 ne contient aucun site de restriction pour les endonucléases PstI, SmaI, BamHI, BstEII et SphI. Ce fragment a été amplifié par PCR. L'ADN génomique de M. tuberculosis a été digéré à l'aide de ces enzymes, et sondé avec le fragment de PCR marqué au 32P. Comme on peut le voir sur la fig. 5, une bande seulement a été observée lorsque l'ADN génomique a été digéré avec chacune des cinq enzymes, ce qui suggère que le gène est présent en une seule copie dans le génome de M. tuberculosis.

### D'autres fusions PhoA portant les séquences signal putatives

La fig. 4 C montre la séquence d'une insértion portée par un vecteur recombinant (pExp59) fusionné avec phoA. Elle présente une séquence signal typique permettant l'exportation de protéines. La séquence présentée est conforme aux règles habituelles telles qu'établies chez les bactéries Gram négatif (25). Elle contient deux acides aminés chargés positivement (Arg, Asn) après le codon de départ, suivis d'un peptide hydrophobe, avec un Gly, correspondant probablement à une boucle dans la structure tridimensionnelle du peptide. Un site potentiel de clivage par la signal peptidase est indiqué par une flèche, ce qui donne une protéine de fusion avec un poids moléculaire proche de celui de phoA, comme le montre la fig. 3, colonne 8, conformément.

### Protéines de fusion PhoA avec des motifs d'acides aminés conservés avec des stéaroyle-protéine-porteuse-d'acyle(ACP) désaturases

Les ACP-désaturases sont des enzymes impliquées dans les voies de la biosynthèse des acides gras. En général, ces enzymes sont des protéines membranaires intégrales (29). Des analyses du plasmide pExp421 de la banque préparée ont révélé deux motifs d'acides aminés conservés avec des ACP-désaturases, un de 9 acides aminés et le deuxième de 14 acides aminés (fig. 4 D). Le reste de la séquence n'a révélé aucune similitude significative avec des protéines connues.

### DISCUSSION

Plus de 30 protéines sécrétées ont été trouvées dans des filtrats du BCG ou de M. tuberculosis à court terme, avec un minimum de lyse de la bactérie (1,19,38). Ces protéines ont été classées selon leur poids moléculaire et leurs réactivités immunologiques. Certaines ont été caractérisées de manière plus poussée. Par exemple, les protéines sécrétées du complexe de l'antigène 85 (les antigènes 85 A,B et C) sont des protéines de 32 kDa présentant des réactions sérologiques croisées (7,35). Les antigènes 85 A et 85 B présentent une affinité vis-à-vis de la fibronectine et seraient impliqués dans l'internalisation de M. tuberculosis dans les macrophages. Les gènes de ces protéines immunogènes (7), et de protéines de 23 kDa (MPB64) (37) et de 19 kDa (5) ont été clones et séquences et des séquences de peptides signal caractéristiques de protéines exportées ont été trouvées. Les protéines recombinantes produites à partir de ces gènes seraient des outils intéressants pour le diagnostic sérologique de la tuberculose. La superoxyde dismutase (SOD) de 23/28 kDa est abondante dans les filtrats de culture à court terme, et serait impliquée dans la survie des mycobactéries dans le phagolysosome. Le gène codant pour la SOD chez M. tuberculosis a été cloné et séquencé (39). De manière intéressante, aucune séquence de peptide signal caractéristique n'a été trouvée. Ceci suggère une voie spécifique de sécrétion de cet enzyme par les mycobactéries. Des protéines sécrétées dans deux gammes étroites de poids moléculaire (6-10 kDa et 26-34 kDa) sont des antigènes majeurs des cellules T (3) et induisent chez la souris des réponses immunitaires en cellules T protectrices vis-à-vis d'une épreuve par des mycobactéries vivantes du complexe de M. tuberculosis (4). Il a été suggéré que les différences dans les réponses immunitaires observées entre les bactéries vivantes et tuées sont dues à ces protéines exportées/sécrétées (20). Ces divers résultats préliminaires suggèrent qu'une meilleure caractérisation de protéines exportées/sécrétées des bactéries pathogènes du complexe de M. tuberculosis serait de grande utilité à la fois pour la compréhension de leur pouvoir pathogène et pour la mise au point de nouveaux vaccins.

Si les protéines sécrétées ont été étudiées par des méthodes biochimiques, d'autres méthodologies génétiques s'avéreraient nécessaires. En utilisant un gène phoA tronqué, des systèmes de fusion ont été mis au point permettant la fixation des extrémités amino d'autres protéines sur PhoA. Cette approche est basée sur la phosphatase alcaline bactérienne périplasmique de E. coli. Cette enzyme doit être localisée de manière extracytoplasmique pour être active. Ainsi, la phosphatase alcaline peut être utilisée comme sonde de localisation subcellulaire.

Une méthodologie PhoA a été élaborée et décrite ici pour l'identification de protéines exportées par les mycobactéries. L'insertion de blaF* dans pJEM11 conduit à la production chez M. smegmatis de protéines de fusion avec l'activité phosphatase alcaline. De plus, des fusions PhoA avec 3 fragments différents de BlaF* étaient enzymatiquement actives, ce qui suggère que la plupart des fusions en phase avec des protéines exportées auront une activité PhoA.

Une banque d'insertions de M. tuberculosis dans pJEM11 a été construite et exprimée chez M. smegmatis. Dans cette banque, une partie du gène codant pour la lipoprotéine exportée connue de 19 kDa (pExp410) a été isolée. Cette protéine de M. tuberculosis est l'un des antigènes sérologiquement immunodominants trouvés chez ce bacille. Des analyses de la séquence d'ADN du gène codant pour cet antigène indiquent que la région NH2-terminale hydrophobe est un peptide signal de lipoprotéine (5). Une partie de cette lipoprotéine a été fusionnée à la protéine A de surface externe de Borrelia burgdorferi pour construire un vaccin BCG recombinant capable d'induire une forte réponse immunitaire (31).

Deux autres séquences partageant des similitudes avec les protéines exportées ou membranaires ont également été identifiées:

pExp53 s'est révélé présenter des similitudes avec le gène de l'antigène de 28 kDa de M. leprae. Cet antigène de M. leprae a été trouvé par criblage d'une banque de λgt 11 avec le sérum de patients atteints de la forme lépromateuse de la lèpre. C'est un antigène majeur impliqué dans la réponse immunitaire humorale à M. leprae (9). De manière intéressante, il a été montré qu'un peptide de 20 acides aminés de cette protéine présente une similitude considérable avec un peptide de l'antigène de 19 kDa de M. tuberculosis, et c'est un épitope de cellules T présentant des réactions croisées (12). La séquence d'ADN du gène codant pour l'antigène de 28 kDa de M. leprae suggère que "la séquence d'acides aminés prédite de la protéine contient un peptide signal potentiel à son extrémité amino-terminale et deux longs domaines hydrophobes, ce qui suggère qu'elle est ciblée pour la localisation sur la membrane plasmatique bactérienne ou la paroi cellulaire" (9).

Une protéine de fusion codée par un plasmide de notre banque (pExp421) partageait des motifs d'acides aminés avec les désaturases. Les ACP-désaturases sont des enzymes impliquées dans les voies de la biosynthèse des acides gras. En général, ces enzymes sont des protéines membranaires intégrales (39). Ce résultat suggère que l'on peut avoir isolé une partie d'un gène important dans le métabolisme des lipides chez M. tuberculosis, peut-être impliqué dans la voie de la biosynthèse de la paroi cellulaire lipidique.

Un autre plasmide (pExp59) avec une séquence signal putative caractéristique a été trouvée. -

En conclusion, les résultats présentés démontrent que la technologie de PhoA pour l'identification génétique de protéines exportées peut être adaptée avec succès pour M. tuberculosis. Des criblages préliminaires d'une banque d'insertions donnant des protéines de fusion PhoA ont mis en évidence des séquences présentant des similitudes avec des protéines exportées connues.

### II) Expression de la protéine P28 de M. tuberculosis

Le BCG est un vaccin vivant. C'est le seul vaccin utilisé pour protéger contre la tuberculose. Son efficacité s'est avérée variable suivant les populations vaccinées, allant d'environ 80% en Grande-Bretagne à 0% en Inde. Il apparaît donc indispensable de recherche un vaccin plus efficace. D'autre part, le fait d'utiliser un vaccin vivant pose aujourd'hui des problèmes en raison de l'extension de l'épidémie du SIDA.

Plusieurs travaux ont montré que des antigènes exportés par Mycobacterium tuberculosis, l'agent de la tuberculose, avaient un effet protecteur contre une épreuve par la souche virulente. Les travaux rapportés ici ont consisté à utiliser une méthode génétique pour isoler et étudier les gènes de M. tuberculosis codant pour des protéines exportées. Nous décrivons ici l'isolement et la caractérisation d'un gène codant pour une protéine possédant des homologies avec la protéine de 28kDa de Mycobacterium leprae déjà décrite.

### Méthodologie pour le clonage de gènes codant pour des protéines exportées.

La méthodologie exposée en détails dans la partie I repose sur l'utilisation de fusions traductionnelles avec le gène codant pour la phosphatase alcaline de Escherichia coli, PhoA. De telles protéines de fusion n'ont une activité phosphatase alcaline détectable que si elles sont exportées. Un vecteur plasmidique portant un gène phoÁ dépourvu de son promoteur, de son site d'attachement à l'ARN ribosomal et de sa séquence signale a été construit. A partir de ce vecteur une activité PhoA ne peut être observée qu'après fusion traductionnelle dans le bon cadre de lecteur avec une protéine exportée. Le vecteur, appelé pJEM11 possède une origine de réplication pour E. coli et une autre pour les mycobactéries. Il possède également un marqueur de sélection, le gène de résistance à la kanamycine du transposon Tn905. Un site multiple de clonage précède le gène phoA tronqué.

Une banque d'ADN génomique provenant d'une souche de M. tuberculosis (Mt103) isolée d'un patient tuberculeux, a été construite dans pJEM11 en insérant des fragments d'ADN issus d'une hydrolyse partielle par l'enzyme Sau3a. Les clones sélectionnés ont permis d'identifier un fragment de nucléotide du gène de 28kDa de M. tuberculosis homologue au gène codant pour la protéine de 28kDa de M. leprae.

Chez les malades lépromateux, des anticorps dirigés contre cette protéine de 28kDa sont observés, laissant supposer que cette protéine est un antigène immunodominant. L'hypothèse a été faite que chez M. tuberculosis, la protéine de 28kDa possédant des homologies avec la protéine de 28kDa de M. leprae pourrait être aussi un antigène immunodominant et qu'il pourrait servir à la construction de tests immunologiques spécifiques permettant la détection de l'infection tuberculeuse ou de la tuberculose maladie. Il pourrait peut-être être utilisé pour la construction de vaccins sous unitaires dans différentes préparations vaccinales. De plus, il pourrait être utile comme vecteur pour l'expression d'antigènes chez les mycobactéries pour la construction de vaccins recombinants.

### Clonage et séquençage du gène codant pour une protéine de 28kDa de M. tuberculosis

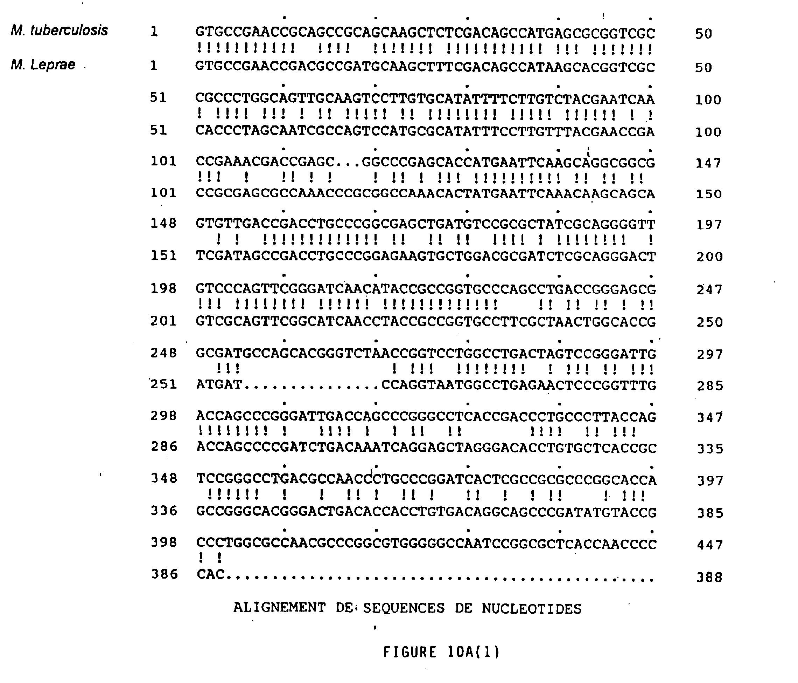

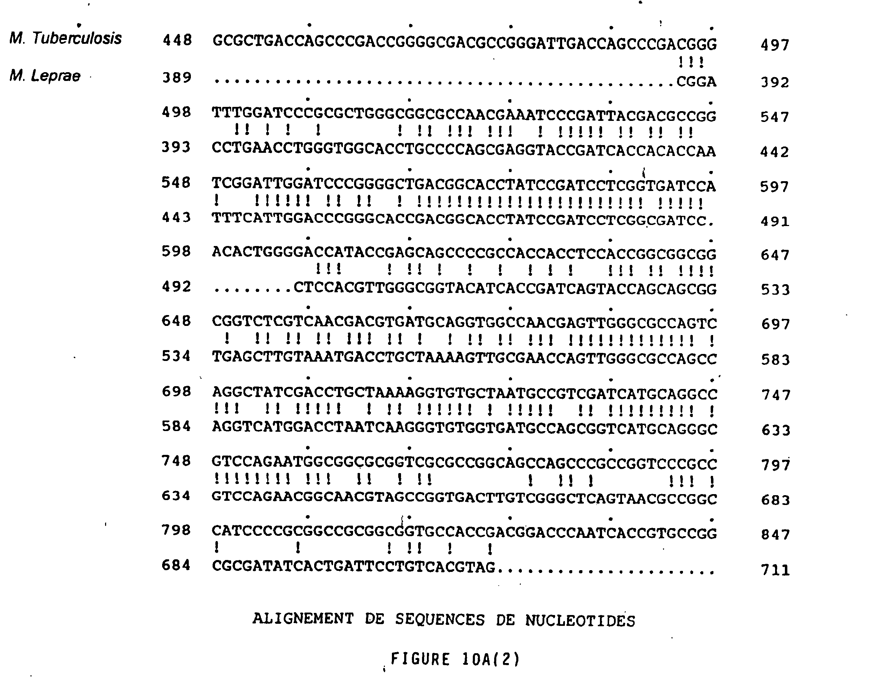

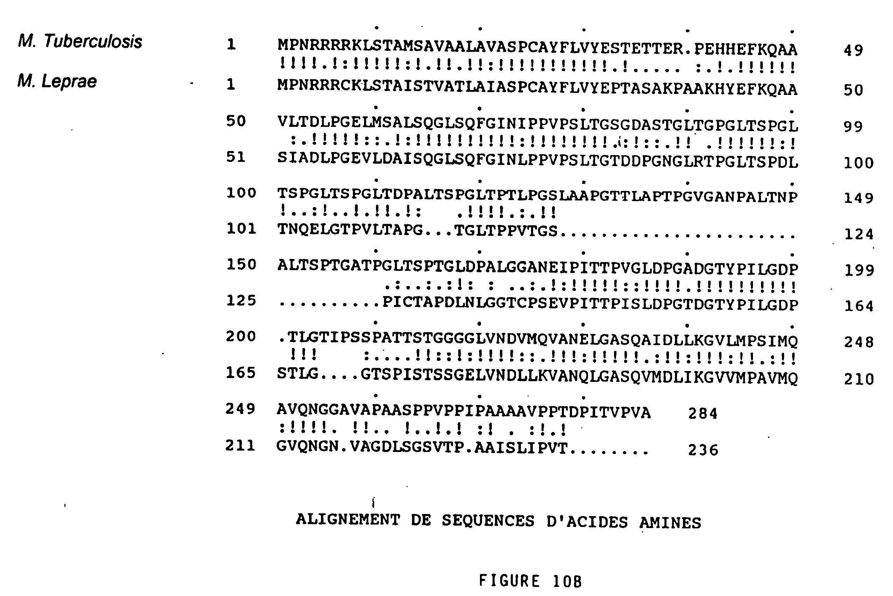

En utilisant l'insertion contenue dans le plasmide pExp53 comme sonde, le gène entier codant pour la protéine de 28kDa de M. tuberculosis a été cloné par hybridation sur colonie d'une banque d'ADN de M. tuberculosis construite en insérant des fragments d'ADN de M. tuberculosis de taille comprise entre 2 et 6 kb obtenus par hydrolyse totale avec l'enzyme PstI dans le vecteur pBluescript KS-. Le fragment PstI de M. tuberculosis correspondant au clone positif et comprenant une insertion de 4,1 kb a été séquencé. La figure 10 montre la séquence nucléotidique du fragment et les similarités avec le gène codant pour la protéine de 28kDa de M. leprae. La séquence du gène de 28kDa de M. tuberculosis est comme celui de M. leprae précédée d'une séquence possédant des similarités avec les boites "fer" retrouvées en amont des gènes exprimés lors d'une carence en fer. Une situation de carence en fer est rencontrée lors de la croissance in vivo. L'hypothèse est faite que l'expression de ce gène est induite lors de la croissance dans les macrophages des mycobactéries hébergeant ce gène. De plus, la protéine de 28kDa de M. tuberculosis possède dans sa partie centrale deux régions contenant des motifs de 5 acides aminés répétés en tandem, qui sont absents de la protéine homologue de M. leprae. Des structures répétées analogues ont été identifiées précédemment dans des antigènes majeurs présents à la surface d'autres agents pathogènes bactériens ou parasitaires tels que la protéine M (40) des Streptococcacea et la protéine CS des Plasmodiae (41).

Tout ou partie de la protéine de 28kDa de M. tuberculosis dont la séquence du gène est présentée ici pourrait être un antigène protecteur potentiel pour la construction d'un vaccin antituberculeux. Un tel antigène peut être obtenu par purification à partir d'extraits cellulaires de M. tuberculosis ou à partir d'extraits cellulaires d'organismes hétérologues génétiquement recombinés. De plus, la protéine de 28kDa de M. tuberculosis ou des peptides en dérivant, pourrait être un antigène utilisable dans des tests ELISA pour le dépistage de malades tuberculeux.

En utilisant le gène de 28kDa de M. tuberculosis comme sonde, une hybridation dans des conditions de stringence élevée n'a été observée qu'avec l'ADN génomique des souches appartenant au complexe M. tuberculosis. En conséquence, la séquence correspondant au gène de 28kDa de M. tuberculosis est une séquence spécifique qui peut être utilisée pour des tests de détection des bacilles de la tuberculose, utilisant des sondes ADN ou ARN et des méthodes d'amplification génique in vitro.

La région régulatrice et le gène de la 28kDa de M. tuberculosis peuvent être utilisés en tant que molécules porteuses pour exprimer des antigènes hétérologues dans le BCG ou tout autre vecteur mycobactérien utile pour la construction de vaccins.

### III) Expression de gènes de mycobactéries ; évaluation de différents promoteurs d'expression

Un aspect important des résultats obtenus concerne la construction d'outils génétiques pour l'étude de l'expression des gènes chez les mycobactéries. Des vecteurs séquence de régulation-sonde ont été utilisés dans l'art antérieur pour isoler et analyser des séquences de régulation chez un grand nombre de bactéries (54). La définition par les inventeurs de tels outils propres aux mycobactéries facilite l'étude des mécanismes génétiques régulant la virulence chez les espèces pathogènes, et l'isolement de nouvelles séquences de régulation qui seraient utiles pour la mise au point de vaccins BCG recombinants améliorés.

Initialement, l'expression de gènes mycobactériens a été étudiée dans des systèmes hétérologues, Escherichia. coli et Streptomyces lividans (46) (51) (60). Ces analyses suggèrent que la plupart des gènes mycobactériens sont plus efficacement exprimés chez S. lividans que chez E. coli. Ultérieurement, des vecteurs à base de plasmides mycobactériens ont été construits qui pourraient être utilisés pour des études dans des systèmes homologues. Les vecteurs pYUB75 et pYUB76 ont été conçus pour sélectionner des fusions de gènes à un gène lacZ tronqué de Escherichia coli (42). Le plasmide pSD7 permet la construction de fusions d'opérons à un gène de la chloramphénicol acétyltransférase (CAT) sans promoteur (47). En utilisant ces vecteurs, un certain nombre de séquences de régulation mycobactériennes ont été isolées et évaluées à la fois chez E. coli et chez Mycobacterium smegmatis.

Les inventeurs ont décrits d'autres constructions de vecteurs de la série pJEM, qui ont plusieurs avantages: ils portent un terminateur de transcription, des multisites de clonages adaptés, et ils permettent des fusions à la fois d'opérons et de gènes à lacZ. lacZ a été choisi comme gène rapporteur car l'enzyme codée, la β-galactosidase, reste active lorsque des séquences hétérologues sont fusionnées à son extrémité amino-terminale (45) (64). Son activité peut être facilement mesurée in vitro, même à des niveaux très bas à l'aide de composés fluorescents (48). La β-galactosidase est également hautement immunogène. Elle induit des réponses immunitaires à la fois humorales et cellulaires après présentation au système immunitaire de souris par des bactéries recombinantes (44) (56). Ainsi, la β-galactosidase peut également être utilisée comme rapporteur du pouvoir immunogène d'un vaccin recombinant. En utilisant des vecteurs pJEM, de nouvelles séquences de régulation actives chez BCG pourraient être isolées et les souches BCG recombinantes facilement testées pour leur capacité à induire des réponses immunitaires chez la souris.

Une étude comparative des activités de divers promoteurs chez M. smegmatis et BCG a également été faite. Les résultats suggèrent que les ARN polymérases de M. smegmatis et de BCG ne partagent pas la même spécificité.

Construction de vecteurs pJEM. Idéalement, un vecteur plasmidique promoteur-sonde doit contenir cinq éléments: i) un réplicon, ii) un marqueur de sélection, et une cassette rapporteur contenant iii) un terminateur de transcription suivi iv) de multisites de clonage (MCS) et v) d'un gène rapporteur dépourvu de ses séquences de régulation.

Pour construire un vecteur de clonage de promoteur les mycobactéries, nous avons utilisé le réplicon dérivé du plasmide pAL5000 de Mycobacterium fortuitum et le gène de la résistance à la kanamycine (aph) de Tn903 (58) ont été utilisés. Ces éléments génétiques sont les composants de base de la plupart des plasmides actuellement utilisés pour la transformation des mycobactéries. Ils semblent conférer une haute stabilité aux clones transformés de M. smegmatis et M. bovis BCG à la fois in vitro et in vivo (chez la souris) même en l'absence de sélection par les antibiotiques (56). Pour faciliter la préparation et la manipulation d'ADN épisomal, la plupart des ces plasmides contiennent également un réplicon de E. coli. Ainsi, nous avons choisi le plasmide pRR3, un vecteur navette E. coli-mycobactéries qui contient ces trois éléments génétiques comme vecteur de base (58).

Aucun terminateur de transcription mycobactérien n'a encore été caractérisé. Pour examiner si le terminateur de transcription du coliphage T4 (tT4) était actif comme site de terminaison pour les ARN polymérases de mycobactéries, l'interposon oméga (57) a été cloné dans le plasmide pJN3, en amont de l'élément sRBS-cII-lacZ, générant pJN11 (fig. 11). Le fragment oméga est composé d'un gène de résistance à la streptomycine/spectinomycine encadré par de courtes répétitions inversées contenant tT4. L'insertion de oméga dans un fragment d'ADN conduit à la terminaison de la synthèse d'ARN chez E. coli (57). pJN3 a été construit en clonant, dans le site ScaI de pRR3, une cassette composée d'un lacZ tronqué associé à un RBS de synthèse (sRBS) et l'extrémité 5' du gène de régulation cII du phage lambda et le promoteur pL (fig. 11). M. smegmatis mc2155 (61) a été transformé avec pJN3 (pL-sRBS-cII-lacZ) ou pJN11 (pL-X-sRBS-cII-lacZ) par électroporation et les clones transformants ont été repérés après croissance sur des boites LB-Xgal. Les clones transformants portant pJN3 ont donné des colonies bleues et les clones transformants portant pJN11 ont donné des colonies blanches. L'activité de la β-galactosidase chez M. smegmatis (pJN11) était 50 fois inférieure à celle chez M. smegmatis (pJN3) (tableau 2). Ainsi, tT4 contenu dans l'insert X agit comme terminateur de transcription efficace chez M. smegmatis.

Un fragment d'ADN contenant le segment tT4 suivi de l'élement sRBS-cII-lacZ de pJN11 a été synthétisé in vitro par amplification par PCR et un MCS (MCS1), contenant 6 sites de restriction uniques, a été ajouté. La cassette résultante a ensuite été clonée dans le site ScaI de pRR3, donnant le vecteur de fusion d'opérons pJEM15 (fig. 12). L'électroporation de M. smegmatis MC²155 et du BCG avec ce plasmide a conduit à des colonies blanches sur des boîtes de LB-Xgal avec une très faible activité de la β-galactosidase (tableau 2). Par contre chez E. coli, pJEM15 a exprimé une activité de la β-galactosidase plus élevée, et par conséquent une coloration bleue sur des boites de LB-Xgal. Ceci est probablement dû à son nombre de copies élevé. Chez E. coli, des vecteurs pUC sont présents en un nombre de copies élevé (supérieur à 500), alors que chez les mycobactéries, les plasmides dérivés de réplicons pAL5000 ont un nombre de copies d'approximativement 3 à 10. (50). Le test de fragments d'ADN pour l'activité promoteur, à l'aide de pJEM15, par criblage bleu-blanc doit ainsi être effectué directement chez les mycobactéries.

Pour obtenir des vecteurs permettant des fusions de gènes à lacZ, nous avons suivi une stratégie similaire. Les trois formes de lacZ tronqué de la série pNM480 (55), qui diffèrent les unes des autres par la "mise en phase traductionnelle" d'un site HindIII localisé à son extrémité 5', ont été clonées, en aval de tT4 et d'un MCS (MCS2) contenant 7 sites de restriction uniques, dans le site ScaI de pRR3. Les plasmides résultants pJEM12-13-14 (fig. 12) permettent ainsi le clonage d'une large gamme de fragments de restriction en phase avec lacZ.

Evaluation de divers promoteurs chez M. smegmatis et BCG. Des fusions d'opérons entre la cassette rapporteur cII-lacZ de pJEM15 et les promoteurs pAN (56), pblaF* (63), psul3 (52), et pgroES/EL1 (49) ont été construits. L'activité de ces promoteurs a été évaluée chez M. smegmatis et chez M. bovis BCG. Les trois premiers promoteurs ont été isolés à partir d'espèces mycobactériennes : pblaF* est un mutant d'expression élevée de pblaF, qui dirige l'expression du gène de la p-lactamase de M. fortuitum ; pAN est un promoteur de M. paratuberculosis et psul3 un composant d'élément génétique mobile de M. fortuitum Tn610. Ces promoteurs ont été localisés sur la base de la cartographie de sites de début de transcription (pblaF* et pAN) ou par analyse de délétion (psul3) (62). pgroES/EL1 est un promoteur de Streptomyces albus qui régule l'expression de l'opéron groES/EL1, et est actif à la fois chez M. smegmatis et le BCG (65).

Les expériences de clonage ont été effectuées directement chez M. smegmatis. Des fragments d'ADN contenant chacun des promoteurs ont été isolés et insérés à MCS1 de pJEM15 digéré par les enzymes de restriction appropriées. Les mélangés de ligation résultants ont été utilisés pour transformer M. smegmatis mc2155 par électroporation et des colonies bleues ont été sélectionnées pour électroduire E. coli MC1061 (45) comme décrit précédemment (43). Les plasmides ont été isolés de ces clones de E. coli et analysés. Ceux correspondant aux constructions recherchées pJN29 à pJN32 (tableau 2), ont été utilisés pour l'électroporation de BCG (souche Pasteur).

L'activité de la β-galactosidase a été dosée sur des extraits soniqués de M. smegmatis et de BCG (tableau 2). L'activité des promoteurs a varié considérablement aussi bien entre les promoteurs chez un hôte mycobactérien et entre les hôtes pour chaque promoteur. La force relative de ces promoteurs n'était pas la même chez M. smegmatis et BCG. Bien que pblaF* ait été le promoteur le plus puissant à la fois chez M. smegmatis et chez le BCG, la situation est différente pour les autres promoteurs : pAN et pgroES/EL1 étaient plus actifs que psu13 chez le BCG, mais chez M. smegmatis, psu13 était plus actif que pAN ou pgroES/Ell.

Das Gupta et ses collaborateurs (47) ont criblé des banques d'ADN de M. smegmatis et de M. tuberculosis pour l'activité promoteur chez M. smegmatis. Ils ont signalé une fréquence de promoteurs 10 à 20 fois plus élevée dans l'ADN de M. smegmatis. De plus, des promoteurs très actifs étaient plus rares dans les banques d'ADN de M. tuberculosis que dans celles de M. smegmatis. Ces auteurs ont suggéré que les promoteurs de M. tuberculosis peuvent avoir divergé considérablement de ceux de M. smegmatis. Les résultats présentés ici suggèrent que la machinerie transcriptionnelle de M. smegmatis et de M. bovis BCG, espèce très apparertée à M. tuberculosis, peut être différente.

En conclusion, la famille de vecteurs construits facilite l'étude de l'expression de gènes chez lep mycobactéries. Une large gamme de fragments peuvent être facilement clonés en phase à lacZ' (fusion de gènes) ou en amont de cII-lacZ (fusion d'opérons) et évalués pour l'activité promoteur par criblage bleu-blanc de transformants mycobactériens sur boites LB-Xgal. L'activité de ces promoteurs peut aussi être mesurée (par dosage de l'activité de la β-galactosidase), leurs séquences déterminées, et leur site de début de transcription cartographié (par analyse d'extension d'amorce) en utilisant "l'amorce universelle" ou des séquences apparentées (53) comme amorce.

### IV) Expression de la protéine ERP sous forme recombinante chez E. coli

La protéine ERP a été exprimée sous forme recombinante chez E. coli et purifiée par chromatographie d'affinité. Deux types de fusions entre ERP et des fragments peptidiques ayant une forte affinité pour des supports chromatographiques spécifiques (Amylose, système MalE; Nickel (Ni²⁺) chélaté, pour le système Histidine) ont été réalisées. Il s'agit de :
- ERP dépourvue de sa séquence signale fusionnée en C-ter avec la protéine liant le maltose (MalE) d'E. coli (MalE-ERP);
- ERP dépourvue de sa séquence signale (ERP(His)₆ ss) ou dans sa totalité (ERP(His)₆), et possédant 6 acides aminés Histidine en C-ter.

Après purification, l'analyse de ces trois protéines de fusion en électrophorèse PAGE-SDS indique que le polypeptide ERP possède un poids moléculaire (PM) relatif de 36 kDa. Il existe une différence importante entre le PM calculé à partir de la séquence (28 kDa) et la PM observé expérimentalement (36 kDa). Ce retard dans la migration électrophorétique pourrait provenir de la forte teneur en résidus Proline, ou de modifications post-traductionnelles.

### REFERENCES

**1.** Altschul, B.F. et al., 1990, J. Mol.. Biol., 215: 403-410.
**2.** Andersen, P. et al., 1991, Infect. Immun. 59: 1905-1910.
**3.** Andersen, P. et al., 1991, Infect. Immun. 59: 1558-1563.
**4.** Andersen, P. et al., 1994, Immun. 62: 2536-2544.
**5.** Ashbridge, K.R. et al., 1989, Nucl. Acid. Res. 17: 1249.
**6.** Boquet, P. et al., 1987, J. Bacteriol. 169: 1663-1669.
**7.** Borremans, M. et al., 1989, Infect. Immun. 57: 3123-3130.
**8.** Brockman, R.W. et al., 1968, Biochemistry 7: 2554-2561.
**9.** Cherayil, B. et al., 1988, J. Immunol. 12: 4370-4375.
**10.** Gaillard, J.-L. et al., 1991, Cell 65: 1127-1141.
**11.** Gutierrez, C. et al., 1989, Nucl. Acids. Res. 17: 3999.
**12.** Harris, D.P. et al., 1991, J. Immunol. 147: 2706-2712.
**13.** Hoffman, C.S. et al., 1985, Proc. Natl. Acad. Sci. USA 82: 5107-5111.
**14.** Isberg, R.R. et al., 1987, Cell 50: 769-778.
**15.** Knapp, s. et al., 1988, J. Bact. 170: 5059-5066.
**16.** Manoil, C. et al., 1990, J. Bacteriol. 172: 515-518.
**17.** Miller, V.L. et al., 1987, cell. 48: 271-279.
**18.** Minton, N.P., 1984, Gène. 31: 269-273.
**19.** Nagal, s. et al., 1991, Infect. Immun. 59: 372-382.
**20.** Orme, I.M., 1988, Infect. Immun. 56: 3310-3312.
**21.** Orme, I.M. et al., 1993, J. Infect. Disea. 167: 1481-1497.
**22.** Pearce, B.J. et al., 1993, Mol. Microbiol. 9: 1037-1050.
**23.** Pearson, W.R. et al., 1988, Proc. Natl. Acad. Sci. USA. 85: 2444-2448.
**24.** Prentki, P. et al., 1984, Gene. 29: 303-313.
**25.** Pugsley, A.P., 1993, Microbiol. Rev. 57: 50-108.
**26.** Ranes, L.G. et al., 1990, J. Bacteriol. 172: 2793-2797.
**27.** Sambrook, J. et al., 1989. Molecular Cloning: a Laboratory Manual, 2nd ed. Cold Spring Harbor Laboratory Press, Cold Spring Harbour, N.Y.
**28.** Sanger, F. et al., 1977, Proc. Natl. Acad. Sci. USA 74: 5463-5467.
**29.** Shanklin, J. et al., 1991, Proc. Natl. Acad. Sci. USA 88: 2510-2514.
**30.** Snapper, S.B. et al., 1990, Mol. Microbiol. 11: 1911-1919.
**31.** Stover, R.C. et al., 1993, J. Exp. Med. 178: 197-209.
**32.** Taylor, R.K. et al., 1987, Proc. Natl. Acad. Sci. USA 84: 2833-2837.
**33.** Taylor, R.K. et al., 1989, J. Bact. 171: 1870-1878.
**34.** Timm, J. et al., 1994, Mol. Microbiol. 12: 491-504.
**35.** Wiker, H.G. et al., 1992, Microbiol. Rev. 56: 648-661.
**36.** Winter, N. et al., 1991, Gene. 109: 47-54.
**37.** Yamaguchi, R. et al., 1989, Infect. Immun. 57: 283-288.
**38.** Young, D.B. et al., 1992, Mol. Microbiol. 6: 133-145.
**39.** Zhang, Y. et al., 1991, Mol. Microbiol. 5: 381-391.
**40.** Hollingstead S. et al., 1986, J.Biol. Chem. 262: 1677-1686.
**41.** Zavala, F. et al;, J. Exp. Med. 157: 194-1957.
**42.** Barletta, R.G. et al., 1992, J. Gen. Microbiol. 138: 23-30.
**43.** Baulard, A. et al., 1992, Nucleic Acids Res. 20: 4105.
**44.** Brown, A. et al., 1987, J. Infect. Dis. 155: 86-92.
**45.** Casabadan, M.J. et al., 1980, J. Bacteriol. 143: 971-980.
**46.** Clark-Curtiss, J.E. et al., 1985, J. Bacteriol. 161: 1093-1102.
**47.** Das Gupta, S.K. et al., 1993, J. Bacteriol. 175: 5186-5192.
**48.** Garcia-del-Portillo, F. et al., 1992, Mol. Microbiol. 6: 3289-3297.
**49.** Guglielmi, G. et al., 1993, Basic and Applied Genetics. Americain Society for Microbiology, Washington, D.C.
**50.** Hatfull, G.H. et al., 1993. Genetic transformation of mycobacteria. TIM 1: 310-314.
**51.** Kieser, T. et al., 1986, J. Bacteriol. 168: 72-80.
**52.** Martin, C. et al., 1990, Nature 345: 739-743.
**53.** Messing, J., 1983, New M13 vectors for cloning, p. 20-78. In R. Wu, L. Grossman and K. Moldave (eds.), Methods in Enzymology. Academic Press, New York.
**54.** Miller, J.H., 1991, Bacterial Genetic Systems, In J.N. Abelson and M.I. Simon (eds.), Methods in Enzymology, Academic Press, San Diego.
**55.** Minton, N.P., 1984, Gene 31: 269-273.
**56.** Murray, A. et al., 1992, Mol. Microbiol. 6: 3331-3342.
**57.** Prentki, P. et al., 1984, Gene 29: 303-313.
**58.** Ranes, M.G. et al., 1990, J. Bacteriol. 172: 2793-2797.
**59.** Bambrook, J. et al., 1989, Molecular cloning: a laboratory manual, 2nd ed. Cold Spring Harbor Laboratory Press, Cold Spring Harbor, N.Y.
**60.** Sirakova, T.D. et al., 1989, FEMS Microbiol. Lett. 59: 153-156.
**61.** Snapper, S.B. et al., 1990, Mol. Microbiol. 4: 1911-1919.
**62. Timm, J. et al**. Unpublished ta.

**Tableau 1**

| Strain/Plasmid | Relevant characteristics | Reference |
|---|---|---|
| *E. coli* XL1-Blue | *supE44 hsdR17 recA1 8yrA46 thi relA1 lac-* F' | 27 |
| *M*. *smegmatis* mc²155 | High-transformant mutant of *M*. *smegmatis* ATCC607 | 30 |
| pRR3 | *E*. *coli*-mycobacteria shuttle vector | 26 |
| pPHO7 | pUC derivative carrying a truncated *phoA* gene | 11 |
| pNM480 | pUC derivative carrying a truncated *lacZ* gene | 18 |
| pJEM11 | *E. coli*-mycobacteria shuttle vector carrying a truncated *phoA* gene | this work |
| pLA71 | pJEM11 in which has been cloned a 1,384 bp fragment from *blaF** | 34, this work |
| pLA72 | pJEM11 in which has been cloned a 1,550 bp fragment from *blaF** | 34, this work |
| pLA73 | pJEM11 in which has been cloned the complete *blaF** | 34, this work |
| pExp410 | pJEM11 in which has been cloned part of the *M*. *tuberculosis* 19 kDa antigen gene | this work |
| pExp53 | pJEM11 in which has been cloned part of a *M. tuberculosis* gene similar to the *M*. *leprae* 28 kDa antigen gene | this work |
| pExp59 | pJEM11 in which has been cloned the signal sequence of a *M*. *tuberculosis* unidentified gene | this work |
| pExp421 | pJEM11 in which has been cloned a *M*. *tuberculosis* gene encoding a protein with amino acids motives similar to desaturases | this work |

## Revendications

1. Séquence de nucléotides issue d'un gène codant pour une protéine exportée de M. tuberculosis, **caractérisée en ce qu'**elle est choisie parmi les séquences suivantes :
■ une séquence IA répondant à l'enchaînement de nucléotides représenté à la figure 6A, ou une séquence IB répondant à l'enchaînement de nucléotides représenté à la figure 6B, ou une séquence codant pour une protéine exportée de M. tuberculosis et hybridant dans des conditions de stringence élevée avec une séquence IA ou IB,
■ une séquence comprenant l'enchaînement de nucléotides IA ou comprenant l'enchaînement de nucléotides IB et codant pour une protéine P28 de M. tuberculosis ayant un poids moléculaire théorique d'environ 28kDa,
■ une séquence comprise dans la séquence IA ou dans la séquence IB et codant pour un polypeptide reconnu par des anticorps dirigés contre la protéine P28 de M. tuberculosis répondant à l'enchaînement d'acides aminés VIIIA représenté à la figure 6A ou à l'enchaînement d'acides aminés VIIIB représenté à la figure 6B.
■ une séquence IV comprenant les séquences régulatrices du gène comprenant la séquence codante IA ou la séquence codante IB, lesdites séquences étant contenues dans les séquences nucléotidiques amont et aval représentées à la figure 7A et à la figure 7B,
■ une séquence V répondant à l'enchaînement compris entre les nucléotides 1 à 72 de la séquence IA ou la séquence IB et correspondant à la séquence signal,
■ une séquence VI répondant à l'enchaînement compris entre les nucléotides 62 à 687 de la séquence IA ou la séquence IB,
■ une séquence VII répondant à l'enchaînement compris entre les nucléotides 688 et 855 de la séquence IA ou la séquence IB.

2. Polypeptide de M. tuberculosis, **caractérisé en ce qu'**il répond à l'enchaînement d'acides aminés VIIIA ou à l'enchaînement d'acides aminés VIIIB représentés respectivement à la figure 6A et à la figure 6B ou **en ce qu'**il comprend cet enchaînement.

3. Polypeptide selon la revendication 2, **caractérisé en ce qu'**il a un poids moléculaire théorique d'environ 28 kDa.

4. Polypeptide selon la revendication 2 ou 3, **caractérisé en ce qu'**il a un poids moléculaire observé d'environ 36 kDa, déterminé par migration électrophonétique en gel de polyacrylamide dénaturant (PAGE-DOS).

5. Polypeptide **caractérisé en ce qu'**il comprend une partie de l'enchaînement VIIIA ou de l'enchaînement VIIIB d'acides aminés selon la revendication 2, et **en ce qu'**il réagit immunologiquement avec des anticorps dirigés contre la protéine P28 de M. tuberculosis répondant à l'enchaînement VIIIA ou à l'enchaînement VIIIB d'acides aminés.

6. Polypeptide selon la revendication 2, 3 ou 4, **caractérisé en ce qu'**il comprend un ou plusieurs des enchaînements suivants : PGLTS, PGLTD, PGLTP, PALTN, PALTS, PALGG, PTGAT, PTGLD, PVGLD.

7. Polypeptide selon l'une des revendication 2 à 4, **caractérisé en ce que** un ou plusieurs des enchaînements suivants : PGLTS, PGLTD, PGLTP, PALTN, PALTS, PALGG, PTGAT, PTGLD, PVGLD
est remplacé par une séquence d'acides aminés d'un épitope ou d'un déterminant antigénique d'un organisme pathogène déterminé.

8. Anticorps monoclonaux ou sérum polyclonal dirigés contre un polypeptide selon l'une quelconque des revendications 2 à 7.

9. Composition pour la détection in vitro ou in vivo d'une infection par M. tuberculosis, **caractérisée en ce qu'**elle comprend un polypeptide selon l'une quelconque des revendications 2 à 7 capable de réagir immunologiquement avec des anticorps formés chez un patient infecté par M. tuberculosis.

10. Composition pour la détection in vitro d'une infection par M. tuberculosis, **caractérisée en ce qu'**elle comprend une séquence de nucléotides contenant au moins 9 nucléotides, issue d'une séquence selon la revendication 1, ou une séquence de nucléotides contenant au moins 9 nucléotides et hybridant dans des conditions stringentes avec l'ADN de M. tuberculosis et n'hybridant pas dans les mêmes conditions avec l'ADN de M. leprae, cette séquence étant une séquence d'ADN ou d'ARN, le cas échéant marquée.

11. Utilisation d'un polypeptide ou d'une séquence d'acides aminés selon l'une quelconque des revendications 2 à 7, en tant que molécule porteuse.

12. Hôte cellulaire procaryote ou eucaryote, **caractérisé en ce qu'**il est transformé par une séquence de nucléotides selon la revendication 1 dans des conditions permettant l'expression de cette séquence et/ou son exposition au niveau de la membrane de l'hôte cellulaire et/ou son exportation et/ou sa sécrétion à partir de la susdite membrane.

13. Hôte cellulaire selon la revendication 12, **caractérisé en ce qu'**il s'agit d'une mycobactérie, par exemple d'une souche de M. smegmatis, M. tuberculosis ou M. bovis.

14. Composition immunogène **caractérisée en ce qu'**elle comprend un polypeptide selon l'une quelconque des revendications 2 à 7 ou un hôte cellulaire selon l'une quelconque des revendications 12 ou 13.

## Claims

1. A sequence of nucleotides from a gene coding for an exported protein of M. tuberculosis, **characterized in that** it is selected from the following sequences:
■ a sequence IA fitting the chain of nucleotides illustrated in Fig. 6A, or a sequence IB fitting the chain of nucleotides illustrated in Fig. 6B, or a sequence coding for an exported protein of M. tuberculosis, and hybridizing under high stringency conditions with a sequence IA or IB,
■ a sequence comprising the chain of nucleotides IA or comprising the chain of nucleotides IB and coding for a protein P28 of M. tuberculosis with a theoretical molecular weight of about 28kDa,
■ a sequence comprised in the sequence IA or in the sequence IB and coding for a polypeptide recognized by antibodies directed against the P28 protein of M. tuberculosis, fitting the chain of amino acids VIIIA illustrated in Fig. 6A or the chain of amino acids VIIIB illustrated in Fig. 6B,
■ a sequence IV comprising the regulatory sequences of the gene comprising the coding sequence IA or the coding sequence IB, said sequences being contained in the upstream and downstream nucleotide sequences illustrated in Fig. 7A and Fig. 7B,
■ a sequence V fitting the chain comprised between the nucleotides 1 to 72 of the sequence IA or the sequence IB and corresponding to the signal sequence,
■ a sequence VI fitting the chain comprised between the nucleotides 62 to 687 of the sequence IA or the sequence IB,
■ a sequence VII fitting the chain comprised between the nucleotides 688 and 855 of the sequence IA or of the sequence IB.

2. A polypeptide of M. tuberculosis, **characterized in that** it fits the chain of amino acids VIIIA or the chain of amino acids VIIIB illustrated in Fig. 6A and Fig. 6B, respectively, and **in that** it comprises this chain.

3. The polypeptide according to claim 2, **characterized in that** it has a theoretical molecular weight of about 28kDa.

4. The polypeptide according to claim 2 or 3, **characterized in that** it has an observed molecular weight of about 36 kDa, determined by electrophoretic migration in denaturating polyacrylamide gel (PAGE-DOS).

5. The polypeptide **characterized in that** it comprises a portion of the chain VIIIA or of the chain VIIIB of amino acids according to claim 2, and **in that** it immunologically reacts with antibodies directed against the P28 protein of M. tuberculosis, fitting the chain VIIIA or the chain VIIIB of amino acids.

6. The polypeptide according to claim 2, 3 or 4, **characterized in that** it comprises one or more of the following chains: PGLTS, PGLTD, PGLTP, PALTN, PALTS, PALGG, PTGAT, PTGLD, PVGLD.

7. The polypeptide according to any of claims 2 to 4, **characterized in that** one or more of the following chains: PGLTS, PGLTD, PGLTP, PALTN, PALTS, PALGG, PTGAT, PTGLD, PVGLD
are replaced with a sequence of amino acids of an epitope or an antigenic determinant of a determined pathogenic organism.

8. Monoclonal antibodies or a polyclonal serum directed against a polypeptide according to any of claims 2 to 7.

9. A composition for the in vitro or in vivo detection of an infection by M. tuberculosis, **characterized in that** it comprises a polypeptide according to any of claims 2 to 7, capable of immmunologically reacting with antibodies formed in a patient infected by M. tuberculosis.

10. A composition for the in vitro detection of an infection by M. tuberculosis, **characterized in that** it comprises a sequence of nucleotides containing at least 9 nucleotides, from a sequence according to claim 1, or a sequence of nucleotides containing at least 9 nucleotides and hybridizing under stringent conditions with the DNA of M. tuberculosis, and not hybridizing under the same conditions with the DNA of M. leprae, this sequence being a DNA or ARN sequence, labeled if required.

11. The use of a polypeptide or a sequence of amino acids according to any of claims 2 to 7, as a carrier molecule.

12. A prokaryotic or eukaryotic cell host, **characterized in that** it is transformed by a sequence of nucleotides according to claim 1 under conditions allowing expression of this sequence and/or its exposure at the membrane of the cell host and/or its exportation and/or its secretion from the aforesaid membrane.

13. The cell host according to claim 12, **characterized in that** this is a mycobacterium, for example a strain of M. smegmatis, M. tuberculosis, or M. bovis.

14. An immunogenic composition **characterized in that** it comprises a polypeptide according to any of claims 2 to 7, or a cell host according to any of claims 12 or 13.

## Patentansprüche

1. Nucleotidsequenz, die aus einem Gen stammt, das für ein exportiertes Protein von M. tuberculosis codiert, **dadurch gekennzeichnet, dass** sie aus den folgenden Sequenzen ausgewählt ist:
■ einer Sequenz IA, die der in Fig. 6A dargestellten Nucleotidkette entspricht, oder einer Sequenz UB, die einer in Fig. 6B dargestellten Nucleotidkette entspricht, oder einer Sequenz, die für ein exportiertes Protein von M. tuberculosis codiert und unter erhöhten Härtebedingungen mit einer Sequenz IA oder IB hybridisiert,
■ einer Sequenz, welche die Nucleotidkette IA umfasst, oder die Nucleotidkette IB umfasst, und für ein Protein P28 von M. tuberculosis codiert, das ein theoretisches Molekulargewicht von ca. 28 kDa hat,
■ einer Sequenz, die in der Sequenz IA oder in der Sequenz IB enthalten ist und für ein Polypeptid codiert, das durch die gegen das Protein P28 von M. tuberculosis gerichteten Antikörper erkannt wird, das der in Fig. 6A dargestellten Aminosäurekette VIIIA oder der in Fig. 6B dargestellten Aminosäurekette VIIIB entspricht,
■ einer Sequenz IV, welche die Regulatorsequenzen des Gens umfasst, das die codierende Sequenz IA oder die codierende Sequenz IB umfasst, wobei diese Sequenzen in den in Fig. 7A und Fig. 7B dargestellten Vorläufer- und Nachläufernucleotidsequenzen enthalten sind,
■ einer Sequenz V, die der Kette entspricht, die unter den Nucleotiden 1 bis 72 der Sequenz IA oder der Sequenz IB enthalten ist und der Signalsequenz entspricht,
■ einer Sequenz VI, die der Kette entspricht, die unter den Nucleotiden 62 bis 687 der Sequenz IA oder der Sequenz IB enthalten ist,
■ einer Sequenz VII, die der Kette entspricht, die unter den Nucleotiden 688 bis 855 der Sequenz IA oder der Sequenz IB enthalten ist.

2. Polypeptid von M. tuberculosis, **dadurch gekennzeichnet, dass** es der Aminosäurekette VIIIA oder der Aminosäurekette VIIIB entspricht, die in Fig. 6A bzw. Fig. 6B dargestellt sind, oder dass es diese Kette enthält.

3. Polypeptid nach Anspruch 2, **dadurch gekennzeichnet, dass** es ein theoretisches Molekulargewicht von ca. 28 kDa hat.

4. Polypeptid nach Anspruch 2 oder 3, **dadurch gekennzeichnet, dass** es ein beobachtetes Molekulargewicht von ca. 36 kDa hat, das durch elektrophoretische Auftrennung in denaturierendem Polyacrylamidgel (PAGE-DOS) bestimmt wird.

5. Polypeptid, **dadurch gekennzeichnet, dass** es einen Teil der Aminosäurekette VIIIA oder der Aminosäurekette VIIIB nach dem Anspruch 2 umfasst, und dass es immunologisch mit den Antikörpern reagiert, die gegen das Protein P28 von M. tuberculosis gerichtet sind, das der Aminosäurekette VIIIA oder der Aminosäurekette VIIIB entspricht.

6. Polypeptid nach Anspruch 2, 3 oder 4, **dadurch gekennzeichnet, dass** es eine oder mehrere der folgenden Ketten umfasst: PGLTS, PGLTD, PGLTP, PALTN, PALTS, PALGG, PTGAT, PTGLD, PVGLD.

7. Polypeptid nach einem der Ansprüche 2 bis 4, **dadurch gekennzeichnet, dass** eine oder mehrere der folgenden Ketten: PGLTS, PGLTD, PGLTP, PALTN, PALTS, PALGG, PTGAT, PTGLD, PVGLD
durch eine Aminosäuresequenz eines Epitops oder eine Antigendeterminante eines bestimmten pathogenen Organismus ersetzt wird.

8. Monoklonale Antikörper oder polyklonales Serum, die/das sich gegen ein Polypeptid nach einem der Ansprüche 2 bis 7 richten/richtet.

9. Zusammensetzung für die *in vitro-* oder *in vivo*-Erfassung einer Infektion durch M. tuberculosis, **dadurch gekennzeichnet, dass** sie ein Polypeptid nach einem der Ansprüche 2 bis 7 umfasst, das in der Lage ist, immunologisch mit den Antikörpern zu reagieren, die sich bei einem durch M. tuberculosis infizierten Patienten gebildet haben.

10. Zusammensetzung zur *in vitro*-Erfassung einer Infektion durch M. tuberculosis, **dadurch gekennzeichnet, dass** sie eine Nucleotidsequenz umfasst, die mindestens 9 Nucleotide enthält, die aus einer Sequenz nach Anspruch 1 stammen, oder eine Nucleotidsequenz, die mindestens 9 Nucleotide enthält und unter Härtebedingungen mit der DNA von M. tuberculosis hybridisiert und unter denselben Bedingungen mit der DNA von M. leprae nicht hybridisiert, wobei diese Sequenz eine gegebenenfalls markierte DNA oder RNA ist.

11. Verwendung eines Polypeptids oder einer Aminosäuresequenz nach einem der Ansprüche 2 bis 7 als Trägermolekül.

12. Prokaryotischer oder eukaryotischer Zellwirt, **dadurch gekennzeichnet, dass** er durch eine Nucleotidsequenz nach Anspruch 1 unter Bedingungen transformiert wird, welche die Expression dieser Sequenz und/oder ihre Exposition im Bereich der Membran des Zellwirts und/oder ihren Export und/oder ihre Sekretion aus der vorgenannten Membran zulässt.

13. Zellwirt nach Anspruch 12, **dadurch gekennzeichnet, dass** es sich um ein Mycobakterium, beispielsweise eines Stamms von M. smegmatis, M. tuberculosis oder M. bovis handelt.

14. Immungenetische Zusammensetzung, **dadurch gekennzeichnet, dass** sie ein Polypeptid nach einem der Ansprüche 2 bis 7 oder einen Zellwirt nach einer der Ansprüche 12 oder 13 umfasst.
